(19) Europäisches Patentamt
European Patent Office
Office européen des brevets

(11) Veröffentlichungsnummer: **0 114 612**
**B1**

# (12) EUROPÄISCHE PATENTSCHRIFT

(45) Veröffentlichungstag der Patentschrift: **23.03.88**

(21) Anmeldenummer: **84100210.8**

(22) Anmeldetag: **11.01.84**

(51) Int. Cl.⁴: **C 07 C 47/46**, C 07 C 49/757, C 07 C 49/753, C 07 C 47/42, C 07 C 45/34, C 07 C 45/27, C 07 C 45/51, C 07 C 45/79, C 07 C 45/67, C 07 C 45/59, C 07 C 45/60

(54) **Racemisches und optisch aktives 3-Hydroxy-alpha-cyclocitral, deren Acetale und optisch aktive 3-Oxo-alpha-cyclocitralacetale sowie Herstellung und Verwendung dieser Verbindungen.**

(30) Priorität: **20.01.83 DE 3301718**
**17.11.83 DE 3341463**

(43) Veröffentlichungstag der Anmeldung: **01.08.84 Patentblatt 84/31**

(45) Bekanntmachung des Hinweises auf die Patenterteilung: **23.03.88 Patentblatt 88/12**

(84) Benannte Vertragsstaaten: **CH DE FR GB LI NL**

(56) Entgegenhaltungen:
**EP-A- 0 000 719**
**US-A- 4 147 672**
**BULL. CHEMICAL SOCIETY OF JAPAN, Band 55, Juni 1982, Seiten 1907-1911; OSAMU TAKAZAWA et al.: "New Synthesis of Megastigma-4,6,8-trien-3-ones, 3-Hydroxy-beta-ionol, 3-Hydroxy-beta-ionone, 5,6-Epoxy-3-hydroxy-beta-ionol, and 3-Oxo-alpha-ionol"**
**HELVETICA CHIMICA ACTA, Band 63, Fasc. 6, Nr. 154, 1980, Seiten 1456-1462, Schweizerische Chemische Gesellschaft; A. RÜTTIMANN et al.: "Synthese von optisch aktiven, natürlichen Carotinoiden und strukturell verwandten Naturprodukten. V. Synthese von (3R,3'R)-, (3S,3'S)- und (3R,3'S;meso)-Zeaxanthin durch asymmetrische Hydroborierung. Ein neuer Zugang zu optisch aktiven Carotinoidbausteinen"**

(73) Patentinhaber: **BASF Aktiengesellschaft**
**Carl-Bosch-Strasse 38**
**D-6700 Ludwigshafen (DE)**

(72) Erfinder: **Schaefer-Luederssen, Ulrich, Dr.**
**Dieselstrasse 2**
**D-6700 Lugwigshafen (DE)**
Erfinder: **Jaedicke, Hagen, Dr.**
**Budapester Strasse 49**
**D-6700 Ludwigshafen (DE)**
Erfinder: **Paust, Joachim, Dr.**
**Ringstrasse 3**
**D-6708 Neuhofen (DE)**
Erfinder: **Eggersdorfer, Manfred, Dr.**
**Weinbietstrasse 22**
**D-6718 Grünstadt (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft 3-Hydroxy-α-cyclocitralderivate der allgemeinen Formel I

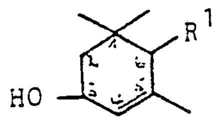

(I)

in der $R^1$ für

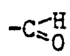 oder 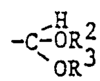

steht, worin $R^2$ und $R^3$ gleich oder verschieden sein können und jeweils eine lineare oder verzweigte Alkylgruppe mit 1 bis 6 C-Atomen bedeuten oder aber $R^2$ und $R^3$ zusammen für eine Ethylengruppe oder eine Propylengruppe stehen, die durch Methylgruppen oder Ethylgruppen substituiert sein können, sowie optisch aktive α-Cyclocitralderivate der allgemeinen Formeln A und B

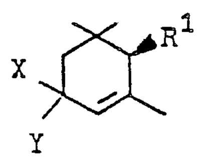

A

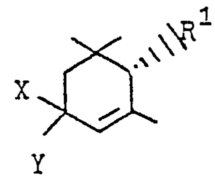

B

in denen $R^1$ für

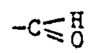

steht, wenn X für Wasserstoff und Y für eine OH-Gruppe steht, oder aber $R^1$ für

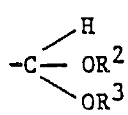

steht, worin $R^2$ und $R^3$ die oben angegebene Bedeutung haben und X und Y zusammen für Sauerstoff stehen oder X für Wasserstoff steht, wenn Y eine OH-Gruppe bedeutet, sowie Verfahren zu deren Herstellung und deren Verwendung zur Herstellung von Safranal und 3-Hydroxy-β-cyclocitral bzw. von optisch aktiven 3-Hydroxy-β-cyclocitralacetalen, die wichtige Zwischenprodukte für die Herstellung von begehrten, naturidentischen optisch aktiven Carotinoiden wie z. B. (3R, 3'R)-Zeaxanthin oder (3R, 3'R, 6'R) Lutein darstellen.

Die genannten Carotinoide sind wichtige natürliche Farbstoffe, die in Pflanzen und Algen vorkommen. Da die Isolierung dieser Farbstoffe aus den natürlichen Vorkommen aufwendig und teuer ist und den Bedarf bei weitem nicht decken kann, wurden zahlreiche Versuche unternommen, diese Verbindungen vollsynthetisch herzustellen. Die Herstellung der dazu benötigten Ausgangsverbindungen mit terpenoiden Strukturelementen, die durch Sauerstoff funktionalisiert sind, war bisher jedoch sehr aufwendig und gelang oft nur in unbefriedigenden Ausbeuten. So beschreiben Loeber et al. in J. Chem. Soc. (C) (1971), Seiten 404-408, einen Syntheseweg zum Zeaxanthin auf folgendem Weg :

Das aus Isophoron hergestellte Keton 1 wird mit einem aus 3-Butin-2-ol hergestellten Grignard-Reagenz zu dem Diol 2 umgesetzt, aus dem durch Abspalten der Ethylendioxy-Schutzgruppe das Dihydroxyketon 3 gebildet wird. Letzteres läßt sich mit Lithiumaluminiumhydrid zu dem Triol 4 reduzieren, welches mit Acetanhydrid in Pyridin zu dem Acetat 5 umgesetzt werden kann. Dehydratisierung von 5 mit Phosphorylchlorid in Pyridin gibt das Enindiacetat 7, welches mittels Lithiumaluminiumhydrid zu dem Enindiol 6 und beim Arbeiten unter strengeren Bedingungen zu dem 3-Hydroxy-β-ionol reduziert werden kann. Selektive Oxidation von 8 mit Mangandioxid führt zu dem 3-Hydroxy-β-ionon 9. Letzteres ergibt beim Umsetzen mit Vinylmagnesiumbromid den Vinylalkohol 10, welcher bei Behandlung mit Triphenylphosphoniumbromid das Wittig-Reagenz 11 bildet, welches beim Erhitzen mit dem Dialdehyd 12 in 1,2-Epoxy-butan Zeaxanthin 14 in guten Ausbeuten ergibt. Kondensation des Wittigsalzes

11 mit ($C_{25}$-)β-Apo-12′-carotenal 16 in 1,2-Epoxybutan liefert β-Cryptoxanthin 18 und mit racemischem ($C_{25}$-)-α-Apo-12′-carotenal das Zeinoxanthin 17. Das folgende Formelschema 1 gibt den beschriebenen Syntheseweg wieder :

(Siehe Formelschema 1 Seite 4 f.)

Wie aus dem Dargelegten hervorgeht, ist die Herstellung der begehrten Carotinoide ausgehend vom 3-Hydroxy-β-ionon 9 recht einfach und vorteilhaft, der Weg zum Hydroxy-β-ionon 9 dagegen durch zahlreiche und aufwendige Reaktionsstufen, wie die mehrfache Umsetzung mit Lithiumaluminiumhydrid und die heterogene Oxidation von 8 mit Braunstein für eine technische Herstellung ungeeignet.

Es war daher die Aufgabe der Erfindung einen neuen vorteilhafteren Weg zur Herstellung von 3-Hydroxy-β-ionon zu finden.

Mit den α-Cyclocitralderivaten der allgemeinen Formel I wurden nun Verbindungen gefunden, die auf relativ einfache Weise zugänglich sind und über die ein neuer technisch vorteilhafterer Zugang zu 3-Hydroxy-β-ionon eröffnet wird. Die 3-Hydroxy-α-cyclocitralacetale der Formel Ia eröffnen außerdem einen neuen vorteilhaften Weg zu dem begehrten Aromastoff Safranal.

Gegenstand der vorliegenden Erfindung sind demnach neben den oben definierten Verbindungen der Formel I und deren Verwendung zur Herstellung von 3-Hydroxy-β-ionon und Safranal auch ein Verfahren zur Herstellung von 3-Hydroxy-α-cyclocitralderivaten der allgemeinen Formel I

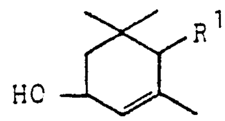

(I)

worin $R^1$, $R^2$ und $R^3$ die eingangs angegebene Bedeutung haben, das dadurch gekennzeichnet ist, daß man

a) das entsprechende Trimethylcyclohexenacetal der allgemeinen Formel II

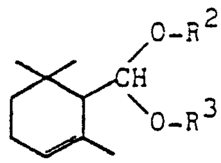

(II)

mit einem geeigneten Oxidationsmittel, vorzugsweise mit Sauerstoff und tert.-Butylhydroperoxid in Gegenwart von Rhodiumverbindungen, insbesondere von Rhodiumtrichloridhydrat oder Rhodiumtristriphenylphosphoniumchlorid oder mit tert.-Butylhydroperoxid in Gegenwart von Cu(II)Acetylacetonat oder mit Di-tert.-butylchromat als geeignetem Oxidationsmittel zu dem 3-Oxo-α-cyclocitralacetal der allgemeinen Formel III

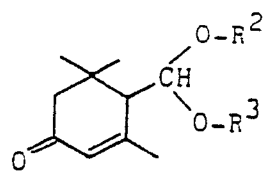

(III)

oxidiert,

b) dieses mit einem geeigneten Reduktionsmittel, vorzugsweise mit Natriumborhydrid in Gegenwart von Cer-(II)-Verbindungen oder mit Di-isobutylaluminiumhydrid oder mit Lithium-tri-tert.-butoxyaluminium-hydrid als geeignetem Reduktionsmittel, zu dem 3-Hydroxy-α-cyclocitralacetal der allgemeinen Formel Ia reduziert

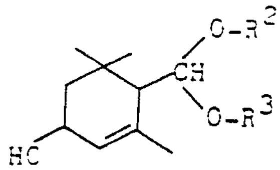

(Ia)

Formelschema 1 :

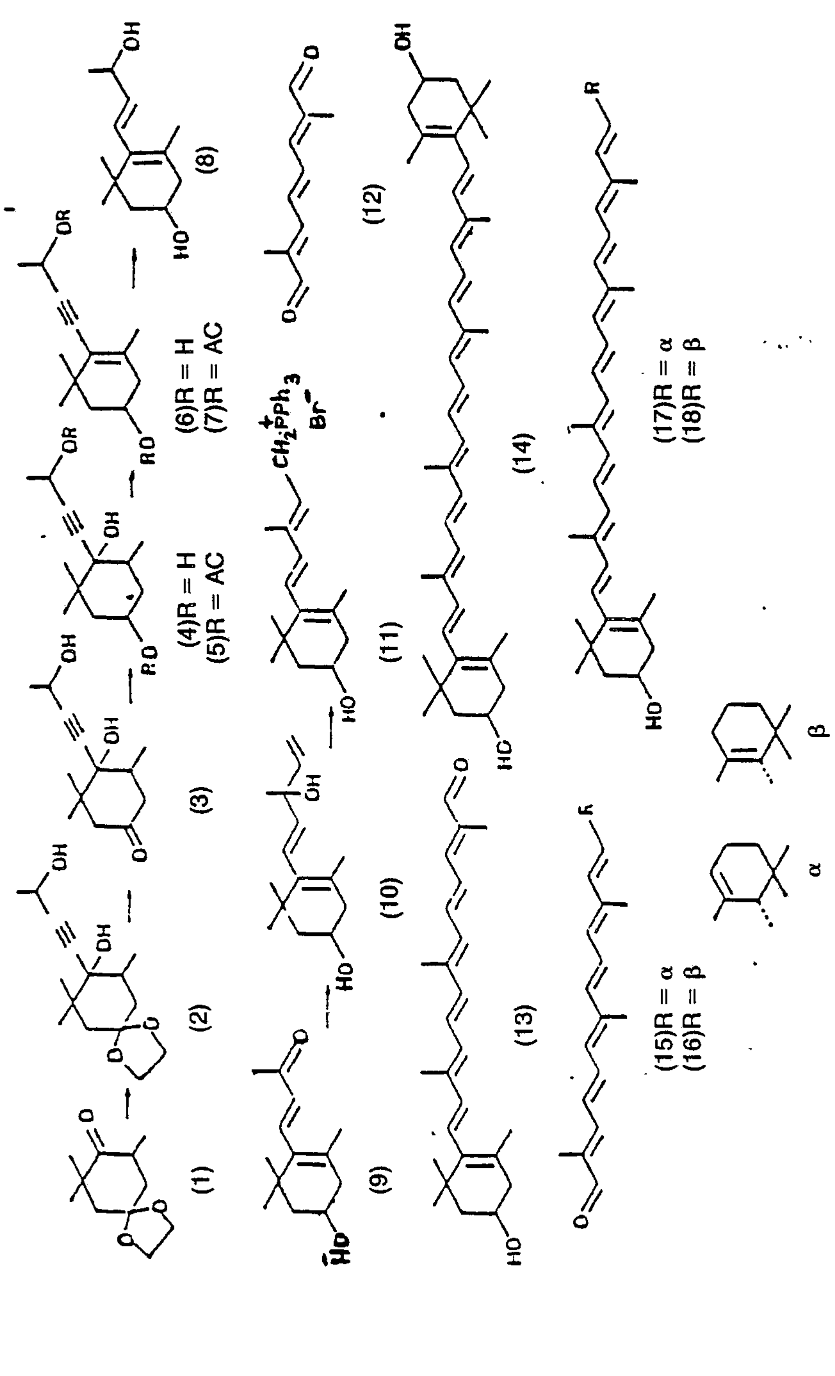

(1) (2) (3) (4)R = H (5)R = AC (6)R = H (7)R = AC (8)

(9) (10) (11) (12)

(13) (14)

(15)R = α (16)R = β (17)R = α (18)R = β

α β

und dieses gegebenenfalls

c) in Gegenwart von organischen oder anorganischen Säuren in wäßrigen organischen Lösungsmitteln, vorzugsweise in Gegenwart von p-Toluolsulfonsäure in etwa 70 (Vol.)-%igem wäßrigem Aceton, hydrolysiert.

Die als Ausgangsstoffe für das erfindungsgemäße Verfahren benötigten Trimethylcyclohexenacetale II sind durch Acetalisierung von α-Cyclocitral unter üblichen Bedingungen, z. B. mit Orthoameisensäuretrimethylester gemäß Bull. Chem. Soc. Jap. 50 (5), S. 1161-1168 (1977) oder mit entsprechenden Glykolen unter Wasserauskreisen in Gegenwart von p-Toluolsulfonsäure mit Toluol als Schleppmittel relativ einfach zugänglich. Somit ergibt sich folgender einfacher Syntheseweg zu dem gewünschten 3-Hydroxy-β-ionon und damit zu Carotinoiden wie z. B. Zeaxanthin :

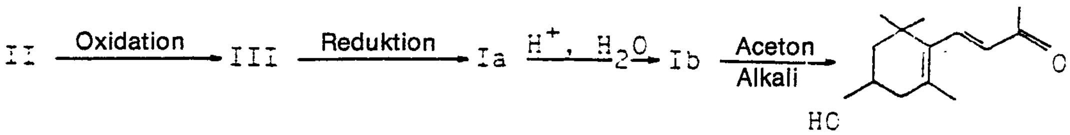


Die als Zwischenprodukte bei dem erfindungsgemäßen Verfahren auftretenden 3-Oxo-α-cyclocitralacetale der allgemeinen Formel III wurden teilweise zwar kürzlich bereits beschrieben (vgl. Takazawa et al., Bull. Chem. Soc. Jap. 55 (1982) Seiten 1907-11), jedoch erscheint in der loc. cit. beschriebene Syntheseweg für diese Verbindungen ausgehend von Isophoron aus mehreren Gründen für ein Verfahren in technischem Maßstab wenig vorteilhaft.

Im folgenden seien die einzelnen Stufen des erfindungsgemäßen Verfahrens näher erläutert.

Zu a)

Die Oxidation der α-Cyclocitralacetale II zu den 3-Oxo-α-Cyclocitralacetalen II läßt sich mit verschiedenen Oxidationsmitteln durchführen. So kann man beispielsweise II mit tert.-Butylhydroperoxid und Sauerstoff oder Luft in Gegenwart von Rhodiumtristriphenylphosphoniumchlorid als Katalysator in III überführen. Das tert.-Butyl-hydroperoxid verwendet man hierbei im allgemeinen in solchen Mengen, daß das Volumenverhältnis von Hydroperoxid zu II 1 : 5 bis 1 : 0,2, vorzugsweise etwa 1 : 1 beträgt. Die Reaktionstemperatur kann zwischen 40 und 110 °C liegen, bevorzugt werden Temperaturen zwischen 60 und 90 °C. Die Umsetzung kann lösungsmittelfrei erfolgen, man kann aber auch das Reaktionsmedium mit einem unter den Reaktionsbedingungen inerten Lösungsmittel, wie tert.-Butanol, Chlorbenzol oder Xylol verdünnen. Die Reaktionsgeschwindigkeit ist bei Verwendung von reinem Sauerstoff gegenüber Luft erhöht, man kann aber das Oxidationsgas auch mit Inertgas verdünnen, falls dies aus Sicherheitsgründen erwünscht ist. Die Katalysatormenge kann ebenfalls in weiten Grenzen variiert werden. Bevorzugt verwendet man Mengen von etwa 1 Mol.%, jedoch auch mit Mengen von 0,1 Mol.% wird das gewünschte Produkt noch erhalten. Eine repräsentative Arbeitsvorschrift findet sich in der Arbeit von Reuter et al (J. Org. Chem 43 (1978) Seiten 2438-42), in welcher die Oxidation verschiedener Cycloalkene beschrieben wird. Darüber hinausgehend wurde überraschend gefunden, daß es bei der erfindungsgemäßen Umsetzung nicht erforderlich ist, den Triphenylphosphinkomplex des Rhodiums einzusetzen, sondern daß man statt dessen $RhCl_3 \cdot 3\,H_2O$ verwenden kann. Unter sonst gleichen Bedingungen kann bei Verwendung von $RhCl_3 \cdot 3\,H_2O$ die Katalysatormenge auf 0,2 bis 0,02 Mol.% gesenkt werden.

Eine weitere Möglichkeit für die Oxidation von II zu III besteht in der Verwendung von tert.-Butylhydroperoxid als Oxidationsmittel und Cu(II)-Acetylacetonat als Katalysator. Auf die Mitverwendung von Luft oder Sauerstoff kann hierbei verzichtet werden, man arbeitet mit Vorteil sogar unter einer Schutzgasatmosphäre.

Hierbei verwendet man das tert.-Butylhydroperoxid im allgemeinen in Mengen von 2 bis 30 Mol, vorzugsweise etwa 10 Mol pro Mol II und das Acetylacetonat in Mengen von 1 bis 100 Mol.%, vorzugsweise etwa 10 Mol.%, bezogen auf II. Die Reaktionstemperaturen können zwischen 40 und 120 °C liegen, die Reaktionszeiten je nach Reaktionstemperatur zwischen 6 und 24 Stunden. Dieses Verfahren ist in der Arbeit von Kimura und Muto (vgl. Chem. Pharm. Bull. Japn. 29 (1981), Seiten 35 bis 42) für die Oxidation von Cholesterylacetat beschrieben.

Die Oxidation von II zu III kann weiterhin mit Di-tert.-butylchromat als Oxidationsmittel durchgeführt werden. Hierbei verwendet man das Di-tert.-butylchromat im allgemeinen in Mengen von 1 bis 10, vorzugsweise etwa 2 bis 4 Mol pro Mol II. Die Umsetzung erfolgt mit Vorteil so, daß man zu einer Lösung von II in Essigsäureanhydrid eine Lösung des Chromats in einem inerten Lösungsmittel wie $CCl_4$ langsam zufügt und das Reaktionsgemisch noch etwa 1 bis 30, vorzugsweise etwa 20 bis 24 Stunden bei Temperaturen von 40 bis 80 °C, vorzugsweise etwa 60 bis 65 °C unter Rühren erhitzt. Dieses Verfahren wird in der Arbeit von Könst und Apeldoorn (vgl. Synth. Commn. 10, Heft 12 (1980), Seiten 899-904) für die Oxidation von α-Ionen beschrieben.

Je nach den Reaktionsbedingungen werden die neuen Cyclohexenone III in Ausbeuten von 30 bis

70 % erhalten.

Es war überraschend, daß unter den beschriebenen Bedingungen mit hoher Selektivität die Wasserstoffatome am Kohlenstoff 3 oxidativ angegriffen werden. Da in II drei verschiedene allylständige Wasserstoffgruppen vorliegen, hätte man die Bildung von komplizierten Produktgemischen erwarten können. Dies umso mehr als auch Reuter et al in loc. cit. unter den entsprechenden Reaktionsbedingungen Doppelbindungsverschiebungen beobachten konnten, die zur Bildung verschiedener Oxidationsprodukte Anlaß gaben. Außerdem konnte man erwarten, daß die Acetalgruppe unter den Reaktionsbedingungen zerstört werden würde. Die entsprechenden gesättigten Acetale zersetzen sich nämlich unter den angegebenen Reaktionsbedingungen in kurzer Zeit unter Bildung von 2,2,6-Trimethyl-cyclohexancarbonsäure (s. Vergleichsbeispiel).

Zu b)

Die Umsetzung der erhaltenen 3-Oxo-α-cyclocitral-acetale III zu den 3-Hydroxy-α-cyclocitralacetalen Ia erfolgt in an sich bekannter Weise, weshalb sich detaillierte Ausführungen erübrigen. Sie kann beispielsweise mit Natriumborhydrid in Gegenwart von Cer(III)-salzen aber auch mit anderen milden Reduktionsmitteln wie Diisobutylaluminiumhydrid oder Lithium-tri-tert.-butoxyaluminiumhydrid in üblicher Weise durchgeführt werden.

Die Alkohole Ia können je nach den Reaktionsbedingungen als cis- oder trans-Form oder auch als Gemisch gebildet werden. Für die weitere Verwendung ist die Isomerenzusammensetzung von Ia aber von untergeordneter Bedeutung.

Zu c)

Die neuen 3-Hydroxy-α-cyclocitralacetale Ia lassen sich mit verdünnten Säuren in mit Wasser mischbaren inerten Lösungsmitteln wie Methanol, Aceton, Tetrahydrofuran leicht zu 3-Hydroxy-α-cyclocitral der Formel Ib hydrolysieren. Wenn man die säurekatalysierte Hydrolyse in wasserfreiem Medium ausführt, erhält man nicht 3-Hydroxy-α-cyclocitral, sondern dessen Dehydratisierungsprodukt Safranal (IV). Aus diesem Grunde muß bei der Hydrolyse von Ia zu Ib das Lösungsmittel 1 bis 80 Gew.%, vorzugsweise 20 bis 70 Gew.% Wasser enthalten. Als Säuren sind hierfür alle nichtoxidierenden mittelstarken bis starken anorganischen oder organischen Säuren geeignet, die üblicherweise für säurekatalysierte Hydrolysen zum Einsatz kommen. Genannt seien beispielsweise Mineralsäuren wie Schwefelsäure oder Phosphorsäure, organische Säuren, wie p-Toluolsulfonsäure oder Citronensäure oder aber saure Kationenaustauscher. Die Menge der Säuren liegt im üblichen Bereich, d.h. zwischen etwa 0,1 und 10 Mol.%, vorzugsweise 0,5 bis 1,5 Mol.%. Die Hydrolyse wird bevorzugt bei Raumtemperatur durchgeführt, sie läuft aber auch bei höheren Temperaturen, wie etwa 50 °C oder tieferen Temperaturen, wie etwa 0 °C ab. Mit besonderem Vorteil arbeitet man mit p-Toluolsulfonsäure in etwa 70 vol.%igem wäßrigem Aceton.

Die Umsetzung von Ia zu Safranal (IV) erfolgt unter den unter c) für die Hydrolyse beschriebenen Bedingungen, jedoch in einem wasserfreien Lösungsmittel.

Die Isomerisierung von 3-Hydroxy-α-cyclocitral (Ib) zu 3-Hydroxy-β-cyclocitral (V) erfolgt in Gegenwart von Alkalihydroxiden, wie NaOH oder KOH oder aber in Gegenwart von Alkalialkoholaten, insbesondere Natriummethylat.

Als geeignete organische Lösungsmittel kommen für diese Isomerisierung zweckmäßigerweise Alkanole, wie Methanol oder Ethanol oder auch Aceton in Betracht. Mit Aceton als Lösungsmittel kann man bei längeren Reaktionszeiten (z. B. 3 h) gleich das Kondensationsprodukt, das 3-Hydroxy-β-ionon isolieren, welches in der von Loeber et al in loc. cit. beschriebenen Weise in das in der Natur vorkommende Carotinoid Zexanthin umgesetzt werden kann.

Die Alkaliverbindungen verwendet man bei diesen Isomerisierungen im allgemeinen in Mengen von 0,5 bis 2 Mol, bezogen auf eingesetztes Cyclocitral. Einen Syntheseweg zum naturidentischen (3R, 3')-Zeaxanthin beschreiben z. B. A. Rüttimann und H. Mayer in Helv. Chim. Acta 63 (1980), Seiten 1456-62 auf folgendem Weg :

Safranal (1) wird mit Diisobutylaluminiumhydrid zum Safranol (2) reduziert und anschließend mit Isopropenylmethylether als Acetal (3) geschützt. Mit dem in situ aus (—)-alph-Pinen und Boran-Dimethylsulfid-Komplex hergestellten chiralen Hydrierungsreagenz (+)-Diisopinocampheylboran kann nach Oxidation mit $NaOH/H_2O_2$ gefolgt von saurer Hydrolyse in 30 %iger Ausbeute das optisch aktive (3R)-3-Hydroxy-β-cyclogeraniol (4) erhalten werden. Mit (+)-alpha-Pinen wird das enantiomere (3S)-3-Hydroxy-β-cyclogeraniol gebildet. Selektive Oxidation von (4) liefert das gewünschte (3R)-3-Hydroxy-β-cyclocitral (5). Umsetzung mit Aceton in Gegenwart von Natriummethylat führt zum (3R)-3-Hydroxy-β-ionon (6), das mit zwei Äquivalenten Vinylmagnesiumbromid das Diol (7) ergibt. Mit methanolischer HCl wird hieraus das Phosphoniumsalz (8) erhalten. Die Wittig-Reaktion mit dem üblichen symmetrischen $C_{10}$-Dialdehyd führt schließlich zum all-E-(3R, 3'R)-Zeaxanthin (9). Die optische Reinheit des Endproduktes wird entscheidend durch die Qualität des als Hilfsreagenz eingesetzten alpha-Pinens bestimmt.

Das folgende Formelschema erläutert diesen Syntheseweg :

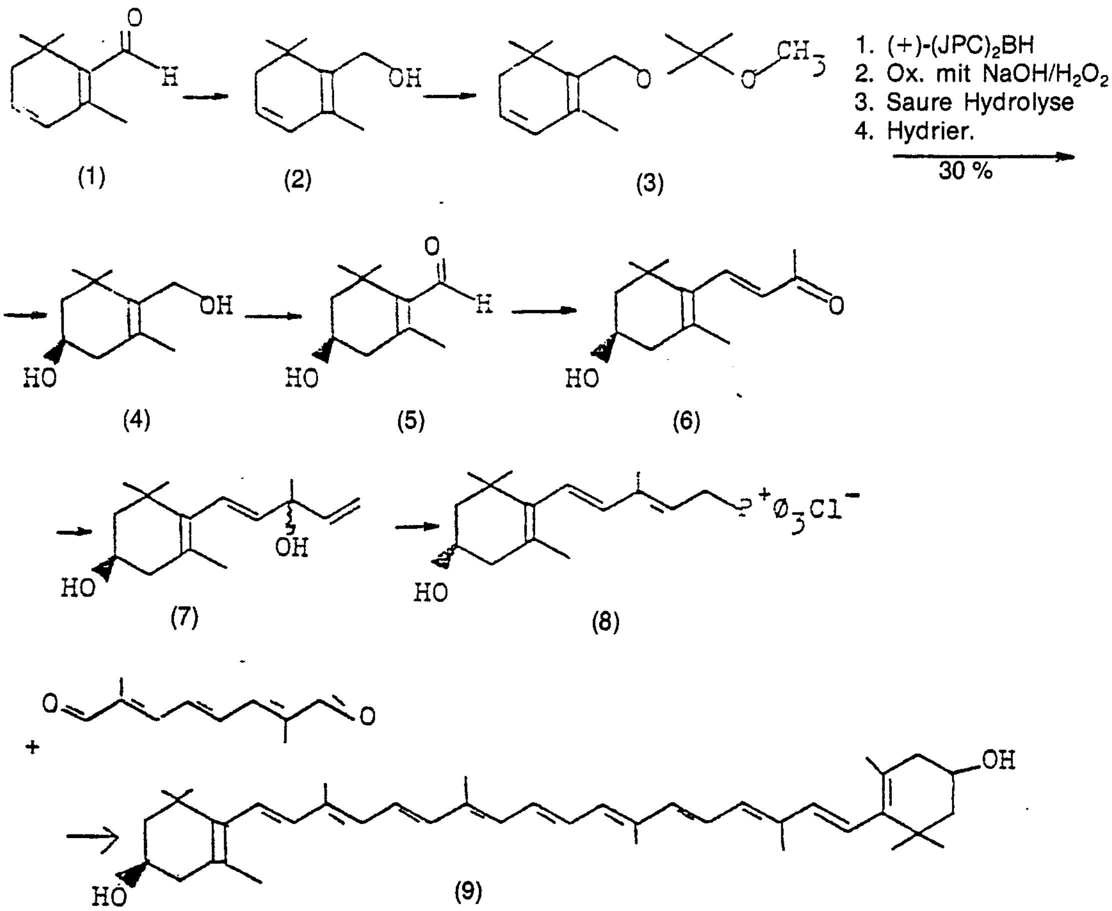


(1) (2) (3)

(4) (5) (6)

(7) (8)

(9)

Wie aus dem Dargelegten hervorgeht, ist die Herstellung des begehrten Carotinoids Zeaxanthin ausgehend von (3R)3-Hydroxy-β-cyclocitral recht einfach und vorteilhaft. Der Weg zum (3R)3-Hydroxy-β-cyclocitral ist jedoch auf dem dargelegten Weg wegen schlecht zugänglicher Ausgangsstoffe und aufwendiger Reaktionsstufen für eine technische Herstellung ungeeignet.

Es war daher die Aufgabe der Erfindung, einen neuen vorteilhafteren Weg zur Herstellung von optisch aktivem 3-Hydroxy-β-cyclocitral zu finden.

Eine Racematspaltung der relativ leicht zersetzlichen 3-Hydroxy-α-cyclocitralacetale oder der freien Aldehyde ließ sich technisch nicht vorteilhaft realisieren.

Da die leicht und in guten Ausbeuten erhältlichen 3-Oxo-α-cyclocitralacetale aufgrund des asymmetrisch substituierten Kohlenstoffatoms (in Position 6) in Form von zwei optisch aktiven Isomeren vorkommen können, könnten optisch aktive 3-Oxo-α-cyclocitralacetale grundsätzlich als Ausgangsverbindungen für das gewünschte optisch aktive 3-Hydroxy-β-cyclocitral eingesetzt werden.

Die Verbindungen besitzen jedoch keine geeigneten funktionellen Gruppen, die eine einfache Trennung in die beiden optisch aktiven Isomeren über diastereomere Hilfsverbindungen zuließen. Aus diesem Grunde mußte nach alternativen Möglichkeiten zur Trennung der optischen Isomeren gesucht werden.

Eine alternative Möglichkeit zur Trennung von optischen Isomeren besteht prinzipiell in der Chromatographie der racemischen Gemische an optisch aktiven Adsorbentien.

Diese optisch aktiven Adsorbentien können aus natürlichen Polymeren, aus derivatisierten natürlichen Polymeren oder aus speziellen synthetischen Polymeren bestehen.

Allerdings lehrt die Literatur, daß als Voraussetzung für eine solche chromatographische Trennung die Möglichkeit der Ausbildung von Wasserstoff-Brückenbindungen (s. G. Blaschke, Angew. Chemie 92 (1980), Seiten 14 bis 25), die Möglichkeit der Ausbildung von Donor-Aceptor-Wechselwirkungen (s. W. H. Pirkle et al., J. Org. Chem. 44 (1979), Seiten 1957 bis 1960) oder das Vorhandensein von möglichst unsubstituierten Phenyl-Resten in der Nähe des Chiralitätszentrums (s. G. Hesse und R. Hagel, Chromatographia 9 (1976), Seiten 62 bis 68) gegeben sein sollten, und deshalb entsprechend substituierte Verbindungen vorliegen müßten.

Überraschenderweise wurde nun gefunden, daß 3-Oxo-cyclocitralacetale durch Chromatographie an solchen optisch aktiven Adsorbentien in ihre optischen Isomeren gespalten werden können.

Es zeigte sich, daß die Trennung an derivatisierter Cellulose, insbesondere an triacetylierter nativer Cellulose, wie sie beispielsweise nach dem sogen. Schering-Verfahren durch Acetylierung von Cellulose

in Benzol mit Perchlorsäure oder in Toluol mit Schwefelsäure als Katalysator (s. Houben-Weyl « Methoden der Organischen Chemie, 4. Aufl., XIV/2, Seite 875, Zeile 5f) erhalten wird, mit niederen Alkanolen als Elutionsmittel zu guten Erfolgen führt. Als Alkanole eignen sich hierfür Methanol, Ethanol, Propanol, iso-Propanol und n- und Isobutanol oder auch Mischungen dieser Alkanole.

Gegenstand der Erfindung ist dementsprechend auch ein Verfahren zur Herstellung von optisch aktiven 3-Oxo-α-cyclocitralderivaten der allgemeinen Formeln A III und B III, das dadurch gekennzeichnet ist, daß man Gemische der entsprechenden racemischen 3-Oxo-α-cyclocitralacetale durch Chromatographie an geeigneten chiralen Adsorbentien, vorzugsweise an derivatisierter Cellulose, in die optisch aktiven Acetale trennt. Mit besonderem Vorteil trennt man die 3-Oxo-α-cyclocitralacetale an triacetylierter Cellulose mit niederen Alkanolen als Elutionsmittel.

Mit Hilfe des oben beschriebenen Verfahrens kann sowohl die rechtsdrehende als auch die linksdrehende Form der Acetale erhalten werden. Wird aus Gründen der Weiterverarbeitung nur die eine der beiden Formen gewünscht, so kann man die unerwünschte Form auf einfache Weise durch Isomerisieren mit Basen in Alkanolen als Lösungsmittel wieder in das Racemat umsetzen.

Das zurückgewonnene Racemat kann wieder der chromatographischen Trennung unterworfen werden. Durch die Rückführung läßt sich schließlich die gesamte Menge an racemischem 3-Oxo-α-cyclocitralacetal in entweder optisch aktives Ia oder Ib überführen. Dieser besondere wirtschaftliche Vorteil wird noch dadurch weiter verstärkt, daß keine teuren Hilfsreagenzien verbraucht werden.

Gegenstand der Erfindung ist weiterhin ein Verfahren zur Herstellung von optisch aktiven 3-Hydroxy-α-cyclocitralderivaten der allgemeinen Formeln A und B

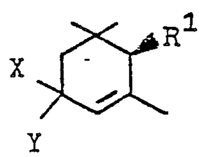

A

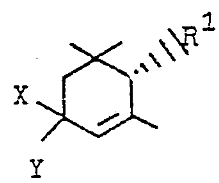

B

worin X und Y sowie $R^1$, $R^2$ und $R^3$ die in Anspruch 1 angegebene Bedeutung haben, das dadurch gekennzeichnet ist, daß man

a) das entsprechende Trimethylcyclohexenacetal der allgemeinen Formel II

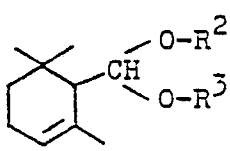

(II)

mit einem geeigneten Oxidationsmittel zu 3-Oxo-α-cyclocitralacetalen der allgemeinen Formel III

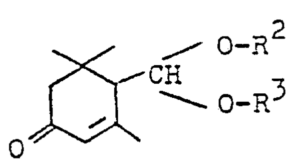

(III)

oxidiert,

aa) diese wie oben beschrieben in die optisch aktiven 3-Oxo-α-cyclocitralacetale der Formeln A III und B III

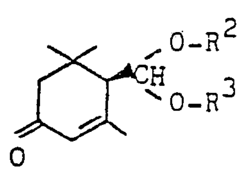

A III

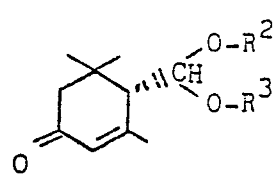

B III

trennt,

b) diese gegebenenfalls mit einem geeigneten Reduktionsmittel stereoselektiv zu optisch aktiven

trans- oder cis-3-Hydroxy-α-cyclocitralacetalen der allgemeinen Formeln A'I und B'I reduziert

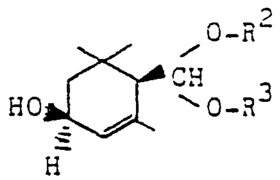
cis-A'I

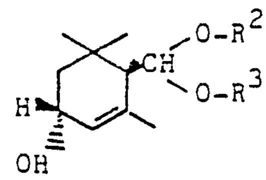
trans-A'I

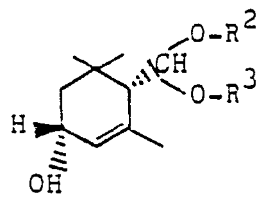
cis-B'I

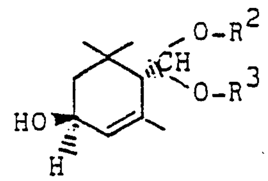
trans-B'I

und diese gegebenenfalls

c) in Gegenwart von organischen oder anorganischen Säuren in wäßrigen organischen Lösungsmitteln zu optisch aktiven 3-Hydroxy-α-cyclocitral der Formeln trans- und cis- A"I und B"I

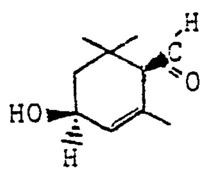
cis-A"I

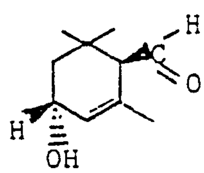
trans-A"I

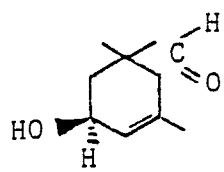
trans-B"I

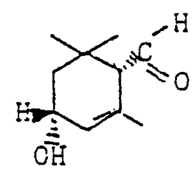
cis-B"I

hydrolysiert.

Als geeignete Reduktionsmittel für den Reaktionsschritt b) seien Natriumborhydrid in Gegenwart von Cer(III)-verbindungen sowie Diisobutylaluminiumhydrid und Lithium-tri-tert.-butoxyaluminiumhydrid genannt. Die bevorzugten Reaktionsbedingungen für die Reaktionsschritte b) und c) sind die gleichen wie sie oben für die Reaktionsschritte b) und c) der entsprechenden racemischen Verbindungen beschrieben wurden.

Gegenstand der Erfindung ist weiterhin die Verwendung der optisch aktiven 3-Hydroxy-α-cyclocitralen der Formeln A"I bzw. B"I zur Herstellung von optisch aktivem 3-Hydroxy-β-cyclocitral der Formeln Va und Vb

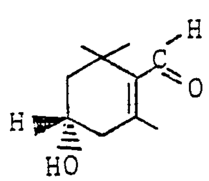
$V^a$

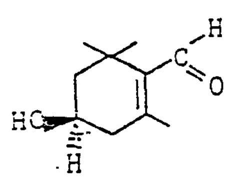
$V^b$

durch Isomerisieren in Gegenwart von Alkalihydroxiden oder Alkalialkoxiden in geeigneten organischen Lösungsmitteln.

## Beispiel 1

Herstellung von 3-Oxo-α-cyclocitral-dimethylacetal

A) Oxidation mit $O_2$ und tert.-Butyl-hydroperoxid in Gegenwart von $RhCl_3 \cdot 3H_2O$

Eine Mischung aus 50 ml (47,6 g ; 0,238 Mol) α-Cyclocitraldimethyl-acetal, 50 ml tert.-Butylhydroperoxid (80 %ig), 200 mg $RhCl_3{\cdot}3H_2O$ und 2 g $NaHCO_3$ wurde bei 60 °C 25 Stunden (h) mit Sauerstoff (5 l/h)

begast. Das Reaktionsgemisch wurde zweimal mit je 40 ml Wasser gewaschen, die Wasserphase mit Methyl-tert.-butylether (MTB) extrahiert, die organischen Phasen vereinigt und getrocknet ($MgSO_4$). In einer Microdünnschichtverdampferapparatur wurde das Rohprodukt bei 0,6 mbar entgast und von leichtsiedenden Anteilen befreit. Der Rückstand (46 g) wurde in 60 g Erdnußöl aufgenommen und bei 0,01 mbar und 130 °C destilliert. Man erhält 36 g eines 71 %igen 3-Oxo-α-cyclocitraldimethylacetals. Das entspricht einer Ausbeute von 50,1 % der Theorie.

B) Oxidation mit (tert.-Butyl)$_2CrO_4$

a) Herstellung von (tert.-Butyl)$_2CrO_4$

Eine Lösung von 138 g (1,38 Mol) $CrO_3$ in 230 ml Wasser wurde bei Raumtemperatur in 246 g tert.-Butanol getropft und das Reaktionsgemisch 1 h bei dieser Temperatur gerührt. Die erhaltene Lösung wurde zweimal mit 700 ml Tetrachlorkohlenstoff extrahiert, die organische Phase mit 250 ml $H_2O$ gewaschen und bei 20 bis 30 °C am Rotationsverdampfer auf ca. 900 ml eingeengt.

b) Oxidation

Zu einer Lösung von 165 g (0,83 Mol) α-Cyclocitraldimethylacetal in 180 ml Essisäureanhydrid wurden innerhalb von 1 1/4 h bei 60 °C 700 ml der gemäß Ba) hergestellten Lösung zugetropft und das Reaktionsgemisch anschließend 22 h bei 60 bis 65 °C gerührt. Nach Abkühlen des Reaktionsgemisches wurde über Kieselgel abgesaugt, mit 1 l $CCl_4$ nachgewaschen und das Filtrat mit 300 ml gesättigter $NaHCO_3$-Lösung neutralgewaschen. Nach dem Trocknen ($MgSO_4$) und Einengen verblieben 169 g Rohprodukt.

Destillation bei 63 bis 80 °C (0,01 mbar) lieferte 68 g eines 95 %igen 3-Oxo-α-cyclocitral-dimethylacetals. Das entspricht einer Ausbeute von 38,5 % der Theorie.

C) Oxidation mit tert.-Butyl-hydroperoxid in Gegenwart von Cu(II)-Acetyl-acetonat.

113,25 g (0,57 Mol) α-Cyclocitraldimethylacetal wurde in 230 ml Toluol gelöst und die Lösung mit 6,9 g Cu(II)-Acetylacetonat versetzt. Das erhaltene Gemisch wurde auf 70 °C erwärmt und mit 283,2 ml tert.-Butylhydroperoxid (80 %ig in Di-tert.-butylperoxid) versetzt. Nach 10 1/2 h wurde das Reaktionsgemisch auf Raumtemperatur (RT) abgekühlt und nach Zugabe von 250 ml Wasser 30 Min. gerührt. Die Phasen wurden getrennt und der organische Anteil mit 200 ml gesättigter Natriumsulfit-Lösung 1 h gerührt. Die wäßrigen Phasen wurden vereinigt, mit 100 ml Hexan extrahiert und die Hexanphase zusammen mit der organischen Phase getrocknet ($MgSO_4$) und bei 45 °C am Rotationsverdampfer eingeengt. Der Rückstand wurde über eine kurze Brücke rasch destilliert ($Kp_{0,05}$ : 90 °C). Die Feindestillation liefert bei einem $Kp_{0.01\ mbar}$ von 80 bis 85 °C 85,5 g 3-Oxo-α-cyclocitraldimethylacetal. Das entspricht einer ausbeute von 70,7 % der Theorie.

Massenspektrum :

m/e = 212 ($M^+$ 0 %) ; 181 (M—$OCH_3$ ; 3 %) ; 137 (M—$CH(OCH_3)_2$ ; 3 %) ; 75 ($CH(OCH_3)2$ ; 100 %)

$^1H$—NMR ($CDCl_3$) δ = 1,046 (3H, S, $C_1$-$CH_3$) ; 1,12 (3 H, S, $C_1$-$CH_3$) ; 1,974 (1H, d, J = 16,5 Hz, $C_2$-Hax) ; 2,10 (3H, S, $C_5$-$CH_3$) ; 2,25 (1H, S, br, $C_6$-H) ; 2,61 (1H, d, J = 16,5 Hz, $C_2$-Heq) ; 3,42 (3H, S, $OCH_3$) ; 3,45 (3H, S, $OCH_3$) ; 4,52 (1H, d, J = 2,1 Hz, $C_7$-H) ; 5,98 (1H, S, $C_4$-H)

## Beispiel 2

Herstellung von 3-Hydroxy-α-cyclocitral-dimethylacetal

A) Reduktion mit Natriumborhydrid

70 g (0,33 Mol) 3-Oxo-α-cyclocitral-dimethylacetal wurden in 600 ml Methanol gelöst und mit 104,5 g $CeCl_3 \cdot 5H_2O$ versetzt. Die Lösung wurde auf 5 °C gekühlt und innerhalb 1 h portionsweise 20 g Natriumborhydrid zugegeben. Anschließend wurde das Gemisch innerhalb von 30 Min. auf RT gebracht und bei dieser Temperatur noch 1 h gerührt. Dann wurde eine Lösung von 40 g Natriumacetat in 800 ml Wasser zugesetzt und das gesamte Reaktionsmedium 5 × mit 400 ml Methylenchlorid extrahiert. Die organische Phase wurde mit 200 ml Wasser gewaschen und mit $MgSO_4$ getrocknet. Nach Abziehen des Lösungsmittels verblieben 71 g eines farblosen Öls, das aus einem Gemisch der cis/trans-Isomeren 80 : 20) von 3 Hydroxy-α-cyclocitraldimethylacetal besteht. Die Ausbeute beträgt 100 % der Theorie.

$^1H$—NMR cis δ =

0,906 (3H, S, $C_1$-$CH_3$) ; 1,01 (3H, S, $C_1$-$CH_3$) ; 1,62 (2H, d, J = 8,1 Hz, $C_2$-$H_2$) ; 1,68 (1H, S, br, OH) ; 1,84 (1H, S, br, $C_6$-H) ; 1,86 (3H, S, br, $C_5$-$CH_3$) ; 3,40 (3H, S, $OCH_3$) ; 3,44 (3H, S, $OCH_3$) ; 4,13 (1H, m, br, $C_3$-H) ; 4,36 (1H, d, J = 2,7 Hz, $C_7$-H) ; 5,61 (1H, S, br, $C_4$-H).

trans δ =

0,936 (3H, S, $C_1$-$CH_3$) ; 1,08 (3H, S, $C_1$-$CH_3$) ; 1,34 (1H, dd, J = 6,48 Hz, J = 13,5 Hz, $C_2$-H) ; 1,86 (3H, S,

$C_5$-$CH_3$) ; 1,90 (1H, dd, J = 6,48 Hz, J = 13,5 Hz, $C_2$-H) ; 2,04 (1H, S, br, $C_6$-H) ; 3,39 (3H, S, $OCH_3$) ; 3,40 (3H, S, $OCH_3$) ; 4,23 (1H, m, br, $C_3$-H) ; 4,28 (1H, S, br, $C_7$-H) ; 5,59 (1H, S, br, $C_4$-H).

B) Reduktion mit Diisobutylaluminiumhydrid

1,27 g (0,005 Mol) 3-Oxo-α-cyclocitraldimethylacetal wurden in 30 ml wasserfreiem n-Hexan gelöst und die Lösung auf 5 °C gekühlt. Bei dieser Temperatur wurden unter $N_2$-atmosphäre innerhalb von 20 Min. 5 ml einer 1 m Lösung von Diisobtylaluminiumhydrid (DIBAH) in Toluol zugetropft und das Reaktionsgemisch noch 30 Min. unter Erwärmen auf RT nachgerührt. Anschließend wurden unter Eisbadkühlung 70 ml Eiswasser zugefügt, die Mischung mit Methyl-tert.-butylether extrahiert, die organische Phase mit Wasser gewaschen, mit $MgSO_4$ getrocknet und am Rotationsverdampfer vom Lösungsmittel befreit. Ausbeute : 0,75 g = 70 % der Theorie.

C) Reduktion mit Tri-tert.-butoxy-lithiumaluminiumhydrid

2,17 g (0,01 Mol) 3-Oxo-α-cyclictraldimethylacetal wurden in 30 ml wasserfreiem Tetrahydrofuran (THF) gelöst und die Lösung mit 5,08 g (0,02 Mol) Tri-tert.-butoxy-lithiumaluminiumhydrid versetzt. Nach 12 h Rühren bei Rückflußtemperaturen wurden 5 ml Methanol tropfenweise zugegeben, das Reaktionsgemisch dann mit 30 ml Wasser verdünnt und mit Methylenchlorid extrahiert. Die organische Phase wurde mit 10 %iger Ammoniumchloridlösung gewaschen, getrocknet und eingeengt. Ausbeute : 1,8 g = 85 % der Theorie.

## Beispiel 3

Herstellung von 3-Hydroxy-α-cyclocitral

Eine Lösung von 15 g (0,07 Mol) 3-Hydroxy-α-cyclocitraldimethylacetal in 360 ml eines 65 %igen wäßrigen Acetons wurde mit 250 mg p-Toluolsulfonsäure versetzt. Nach 12 h Stehen bei Raumtemperatur wurde mit 800 ml Wasser verdünnt. Anschließend wurde 3 × mit 150 ml Diethylether extrahiert und die vereinigten Etherphasen mit 100 ml Wasser gewaschen, dann getrocknet ($MgSO_4$) und am Rotationsverdampfer eingeengt.

Ausbeute 10,30 g ; entsprechend einer Ausbeute von 87,5 % der Theorie.

$^1$H-NMR cis :

δ = 0,952 (3H, S, $C_1$-$CH_3$) ; 1,03 (3H, S. $C_1$-$CH_3$) ; 1,5-1,95 (2H, m, $C_2$-$H_2$) ; 1,64 (3H, S, $C_5$-$CH_3$) ; 2,42 (1H, d, J = 5,4 Hz, $C_6$-H) ; 3,08 (1H, br, OH) ; 4,31 (1H, t, br, J = 7,2 Hz, $C_3$-H) ; 5,02 (1H, S, br, $C_4$-H) ; 9,54 (1H, d, J = 5,4 Hz, $C_z$-H).

trans :

δ = 0,91 (3H, S, $C_1$-$CH_3$) ; 1,10 (3H, S, $C_1$-$CH_3$), 1,5-1,95 (2H, m, $C_2$-$H_2$), 1,64 (3H, S, $C_5$-$CH_3$) ; 2,60 (1H, d, br, $C_6$-H) ; 3,08 (1H, br, OH) ; 4,44 (1H, t, br, J = 5,4 Hz, $C_3$-H) ; 5,02 (1H, S, br, $C_4$-H) ; 9,51 (1H, d, J = 5,4 Hz, $C_7$-H).

## Beispiel 4

a) Oxidation

Analog zu Beispiel 1B wurden 33 g (0,168 Mol) α-Cyclocitralethylenglykolacetal mit 125 ml einer 2,2 m tert.-Butylchromatlösung im Verlauf von 30 h oxidiert. Der Umsatz betrug 47 %. Nach Destillation bei 0,3 mbar und 95 bis 100 °C erhielt man 12,9 g eines gelben Öls, das beim Abkühlen kristallisierte. Die Ausbeute betrug 78 % 3-Oxo-α-cyclocitralethylenglykolacetal, bezogen auf umgesetztes α-Cyclocitralethylenglykolacetal. Nach Umkristallisation aus Diisopropylester betrug der Schmelzpunkt 71 bis 71,5 °C.

Massenspektrum :

m/e : 224 ($M^+$, 5 %), 87 ( 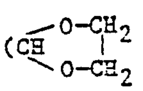 100 %)

$^1$H-NMR

δ = 1,05 (3H, S, $C_1$-CH-3), 1,18 (3H, S, $C_1$-$CH_3$) ; 1,99 (1H, d, J = 17,2 Hz, $C_2$-Hax) ; 2,09 (3H, S, $C_5$-$CH_3$) ; 2,36 (1H, d, J = 2,7 Hz ; $C_6$-H) ; 2,70 (1H, d, J = 17,2, $C_2$-Hequ.) ; 3,76-3,94 (4H, m, $C_4'$, $C_5'$-$H_2$) ; 5,23 (1H, d, J = 27 ; C-H) ; 5,97 (1H, Sbr ; H, $C_4$-H).

b) Reduktion

3,5 g (0,016 Mol) 3-Oxo- -cyclocitralethylenglykolacetal wurden analog Beispiel 2 mit 1 g $NaBH_4$ und 5,2 g $CeCl_3 \cdot 7H_2O$ reduziert. Nach der üblichen Aufarbeitung erhielt man 3,35 g (96 % der Theorie) eines farblosen, hochviskosen Öls, das 83 % trans und 17 % cis-3-Hydroxy-α-cyclocitralethylenglykolacetal

enthält.

$^1$H-NMR für den trans-Alkohol :

δ = ($CDCl_3$) = 0,92 (3H, S, $C_1$-$CH_3$) ; 1,04 (3H, S, $C_1$-$CH_3$) ; 1,646 (2H, d, J = 8 Hz, $C_2$-$H_2$) ; 1,834 (3H, S, $C_5$-$CH_3$) ; 1,92 (1H, brS, $C_6$-H) ; 1,96 (1H, S, br, OH) ; 3,74-4,04 (4H, m, $CH_2$-$CH_2$) ; 4,13 (1H, br, t, J = 8 Hz, $C_3$-H) ; 5,02 (1H, d, J = 1,6 Hz, $C_4$-H) ; 5,62 (1H, S, br, $C_5$'-H).

c) Hydrolyse

1 g (0,0047 Mol) 3-Hydroxy-α-cyclocitralethylenglykol acetal wurde in einer Mischung aus 20 ml 65 vol.%igem wäßrigem Aceton und 10 mg p-Toluolsulfonsäure 12 h bei 45 °C gerührt. Anschließend wurde das Reaktionsgemisch mit 40 ml Wasser verdünnt, mit MTB extrahiert, die organische Phase mit Wasser gewaschen, getrocknet und unter vermindertem Druck eingeengt.

Ausbeute : 0,65 g 3-Hydroxy-α-cyclocitral = 83 % der Theorie.

## Beispiel 5

a) Oxidation

50 g (0,238 Mol) α-Cyclocitralpropylenglykolacetal wurde mit 50 ml tert.-Butylhydroperoxid und 125 mg $RhCl_3 \cdot 3H_2O$ gemischt und bei 95 °C innerhalb von 3 h mit 15 l Sauerstoff begast. Nach dem Abkühlen wurde das Reaktionsgemisch mit 250 ml Methylenchlorid verdünnt und 3 × mit je 25 ml Wasser gewaschen. Die organische Phase wurde mit Natriumsulfat getrocknet und am Rotationsverdampfer eingeengt. Der Rückstand wurde nach dem Entgasen bei 0,63 mbar und 90 °C an einer Molekulardestillationsapparatur destilliert. Man erhielt 21,3 g 3-Oxo-α-cyclocitral-1,3-propylenglykolacetal in Form eines viskosen Öls, das nach Reinigung mit MTB kristallisierte. Fp. 63-65,5 °C ; Ausbeute 38 % der Theorie.

Massenspektrum

m/e : 224 ($M^+$, 5 %) ; 87 (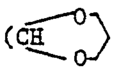, 100 %).

H-NMR :

δ ($CDCl_3$) 1,02 (3H, S, $C_1$-$CH_3$) ; 1,14 (3H, S, $C_1$-$CH_3$) ; 1,31 (1H, d, br, J = 13,5 Hz, $C_5$'-Hax) ; 1,95 (1H, d, J = 16,5 Hz, $C_2$-Hax) ; 2,03 (1H, m, $C_5$'-Heq), 2,11 (3H, S, $C_5$-$CH_3$) ; 2,17 (1H, S, br, $C_6$-H) ; 2,65 (1H, d, J = 16,5 Hz, $C_5$-Heq) ; 3,73 (2H, m, $C_6$'-$H_2$ax) ; 4,11 (2H, m, $C'_{4,6}$—$H_2$, eq) ; 4,88 (1H, d, J = 2,7 Hz, $C'_2$-H) ; S, 97 (1H, S, br, $C_4$-H).

b) Reduktion

Analog Beispiel 2 A wurden 7,1 g (0,032 Mol) 3-Oxo-α-cyclocitral-1,3-propylenglykolacetal mit 1,68 g $NaBh_4$ in Gegenwart von 8,68 g $CeCl_3 \cdot 7H_2O$ umgesetzt.

Ausbeute 7,1 g 3-Hydroxy-α-cyclocitral-1,3-propylenglykolacetal in Form eines cis/trans-Gemisches (~ 15 : 85). Das entspricht einer Ausbeute von 99 % der Theorie.

Massenspektrum :

m/e : 226 ($M^+$, 4 %) ; 87 (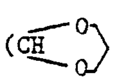, 100 %)

H-NMR für die trans-Verbindung :

δ($CDCl_3$) 0,896 (3H, S, $C_1$-$CH_3$) ; 1,03 (3H, S, $C_1$-$CH_3$) ; 1,31 (1H, d, br, J = 13,0 Hz, $C'_5$-Hax) ; 1,594 (2H, d, J = 8 Hz ; $C_2$-$H_2$) ; 1,764 (1H, S, br, $C_{6\text{-}H)}$ ; 1,880 (3H, S, $C_5$-$CH_3$) ; 1,94 (1H, S, OH) ; 1,95-2,14 (1H, m, $C'_5$-Heq) ; 3,60 (2H, dd, J = 13,0 Hz, J = 27 Hz, $C'_{4,6}$-$H_2$ax) ; 4,11 (2H, m, br, $C_{4,8}$-$H_2$eq) ; 4,15 (1H, m, br, $C_3$-H) ; 4,75 (1H, d, J = 1,6 Hz, $C'_1$-H) ; S, 626 (1H ; S, br, $C_4$-H)

## Beispiel 6

a) Oxidation

Analog zu Beispiel 1 B b wurden 36 g (0,151 Mol) α-Cyclocitralneopentylglykolacetal mit 125 ml einer 2,2 molaren tert.-Butylchromatlösung oxidiert. Nach 30 h waren 72 % des Ausgangsmaterials umgesetzt. Durch Destillation des erhaltenen Rohproduktes bei 0,25 mbar und 115 °C wurden 16,4 g eines hochviskosen Öls erhalten, das 88 % 3-Oxo-α-cyclocitralneopentylglykolacetal enthielt. Aus Diisopropylether kristallisierte das reine 3-Oxo-α-cyclocitralneopentylglykolacetal mit einem Schmelzpunkt von 79 bis 81 °C ; die Ausbeute betrug 60 %, bezogen auf umgesetztes Cyclocitralacetal.

H-NMR δ ($CDCl_3$) :

0,70 (3H, S, $C'_5$-CHeq.) ; 1,028 (3H, S, $C_1$-$CH_3$) ; 1,096 (3H, S, $C'_5$-$CH_3$ax) ; 1,124 (3H, S, $C_1$-$CH_3$) ; 1,94 (1H, d, J = 17,8 Hz ; $C_2$-Hax) ; 2,136 (3H, S, $C_5$-$CH_3$) ; 2,67 (1H, d, J = 17,8 Hz, $C_2$-Heq) , 3,4 (1H, d, J = 10

Hz ; $C'_{4,6}$-H) ; 3,41 (1H, d, J = 10 Hz, $C'_{4,6}$-H) ; 3,57 (1H, d, J = 10 Hz, $C'_{4,6}$-H) ; 3,62 (1H, d, J = 10 Hz, $C'_{4,6}$-H) ; 4,79 (1H, S, $C'_2$-H) ; 5,99 (1H, S, $C_4$-H).

b) Reduktion

4,2 g (0,016 Mol 3-Oxo-α-cyclocitralneopentylglykolacetal wurden analog Beispiel 2 A mit 1 g $NaBH_4$ und 5,2 g $CeCl_3 \cdot 7H_2O$ reduziert. Nach der üblichen Aufarbeitung wurden 4,0 g 3-Hydroxy-α-cyclocitral-neopentylglykolacetal in Form eines farblosen hochviskosen Öls enthalten. Ausbeute : 95 % der Theorie.

$^1$H-NMR δ ($CDCl_3$) :

0,706 (3H, S, $C'_5$-$CH_3$eq) ; 0,90 (3H, S, $C_1$-$CH_3$) ; 1,026 (3H, S, $C_1$-$CH_3$) ; 1,16 (3H, S, $C'_5$-$CH_3$ax) ; 1,592 (2H, d, br, J = 8,6 Hz, $C_2$-$H_2$) ; 1,82 (1H, S, $C_6$-H) ; 1,86 (1H, S, br, OH) ; 1,90 (3H, S, $C_5$-$CH_3$) ; 3,40 (2H, d, br, J = 10,8 Hz, $C'_{2,6}$-$H_2$) ; 3,62 (2H, d, J = 10,8 Hz, $C'_{4,6}$-$H_2$) ; 4,11 (1H, t, br, J = 8,6 Hz, $C_3$-H) ; 4,62 (1H, d, J = 1,5, $C'_2$-H) ; 5,608 (1H, S, br, $C_4$-H).

## Beispiel 7

Verwendung von 3-Hydroxy-α-cyclocitral zur Herstellung von 3-Acetoxy-β-ionon

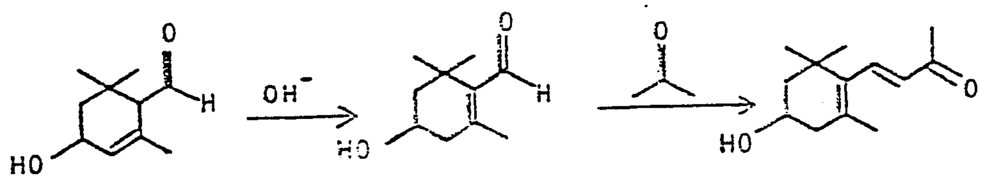


10,3 g (0,061 Mol) 3-Hydroxy-α-cyclocitral wurden mit 5 ml Aceton verdünnt und 8,5 g NaOH-Plätzchen zugegeben. Nach einminütigem Rühren wurden 250 ml Aceton zugegeben und das Reaktionsgemisch 3 Stunden bei Raumtemperatur gerührt. Anschließend wurde die rotbraune Reaktionsmischung mit 750 ml Wasser und mit 100 ml $CH_2Cl_2$ versetzt und die wäßrige Phase noch 3 mal mit je 100 ml $CH_2Cl_2$ extrahiert. Die vereinigten organischen Phasen wurden mit 100 ml 0,2 n HCl sowie 100 ml Wasser gewaschen und mit $MgSO_4$ getrocknet. Nach dem Einengen am Rotationsverdampfer bei 15 mbar und 40 °C verblieben 25,5 g eines honigfarbenen Öls, das 48,7 % an 3-Hydroxy-β-ionon enthält, entsprechend einer Ausbeute von 97 % der Theorie.

Das erhaltene Öl wurde in 140 ml Pyridin gelöst und mit 28 ml Acetanhydrid versetzt. Nach Zugabe von 50 mg 4-Dimethylamino-pyridin wurde 16 Stunden bei Raumtemperatur belassen. Dann wurde die Mischung mit 500 ml Wasser verdünnt und mit verdünnter Salzsäure auf pH 3 gebracht, mit 200 ml $CH_2Cl_2$ extrahiert und 2 × mit 50 ml Wasser, 1 × mit 50 ml gesättigter $NaHCO_3$-Lösung und 1 × mit 50 ml Wasser gewaschen. Nach dem Trocknen über $MgSO_4$ und Einengen am Rotationsverdampfer verblieben 16,23 g 3-Acetoxy-β-ionon (Gehalt 89 %). Das entspricht einer Ausbeute von 94 % der Theorie, bezogen auf eingesetztes 3-Hydroxy-α-cyclocitral.

## Vergleichsbeispiel

11,9 g (0,06 Mol) 2,2,6-Trimethyl-cyclohexan-1-carbaldehyddimethylacetal wurden analog Beispiel 1A oxidiert. Nach 2 Stunden war gaschromatographisch kein Ausgangsmaterial mehr nachzuweisen. Das erhaltene Produkt wurde 2 × mit 30 ml Wasser gewaschen, die vereinigten Wasserphasen 3 × mit 30 ml MTB extrahiert, die organischen Phasen vereinigt, mit $MgSO_4$ getrocknet und am Rotationsverdampfer eingeengt.

Aus Pentan erhält man farblose Kristalle vom Schmelzpunkt 87 bis 90 °C.

NMR δ =

0,9 (3H, d, J = 5,4 Hz, $C_5$-$CH_3$) ; 1,00 (3H, S, $C_1$-$CH_3$) ; 1,05 (3H, S, $C_1$-$CH_3$) ; 1,08-1,27 (m) ; 1,36-1,59 (m) ; 1,68-1,9 (m) ; 11,0-11,3 (1H, S, br, $CO_2$H).

Massenspektrum :

m/e : 170 ($M^+$, 40 %) ; 152 (68 %), 110 (100 %).

Es handelte sich nicht um 3-Oxo-2,2,6-trimethyl-cyclohexan-1-carbaldehyddimethylacetal, sondern um 2,2,6-Trimethyl-cyclohexan-1-carbonsäure.

## Beispiel 8

a) Eine Lösung von 200 mg (0,89 mmol) (±)-3-Oxo-α-cyclocitralpropylenglykolacetal in 2 ml Ethanol wurde auf eine mit 220 g Cellulosetriacetat (hergestellt nach Houben-Weyl « Methoden der organischen Chemie », 4. Auflage, Band 14/2, Seite 875, Zeile 5 f.) gefüllte Glassäule (d = 25,4 mm ; 1 = 1090 mm) aufgetragen und mit 96 %igem Ethanol eluiert (Fließgeschwindigkeit 65 ml/h ; p = 3,5 bar). Das Eluat wurde so fraktioniert, daß etwa 25 % der eingesetzten Menge eine Fraktion mit rechtsdrehenden Antipoden, etwa 50 % eine Racemat-Mittelfraktion und etwa 25 % eine Fraktion mit linksdrehenden Antipoden bildete. Nach Abdampfen des Elutionsmittels am Rotationsverdampfer erhielt man die optisch

aktiven Ketone in einer optisch aktiven Reinheit von 98 bis 100 %

(+)-(6S)-3-Oxo-α-cyclocitralpropylenglykolacetal
$[\alpha]_D^{20}$ = + 147,6° (c = 1 ; $CHCl_3$)

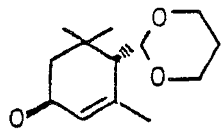

(—)-(6R)-3-Oxo-α-cyclocitralpropylenglykolacetal
$[\alpha]_D^{20}$ = — 152,9° (C = 1 ; $CHCl_3$)

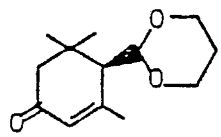

Analog zu dem in Beispiel 1a beschriebenen Verfahren wurden noch folgende optisch aktive Ketone der Formel I erhalten :

b) (+)-(6S)-3-Oxo-α-cyclocitraldimethylacetal
$[\alpha]_D^{20}$ = + 179,0° (C = 1 ; $CHCl_3$)
(—)-(6R)-3-Oxo-α-cyclocitraldimethylacetal
$[\alpha]_D^{20}$ = — 177,2 (C = 1 ; $CHCl_3$)

c) (+)-(6S)-3-Oxo-α-cyclocitralethylenglykolacetal
$[\alpha]_D^{20}$ = + 141,4° (C = 0,4 ; $CH_3OH$)
(—)-(6R)-3-Oxo-α-cyclocitralethylenglykolacetal
$[\alpha]_D^{20}$ = — 139,7° (C = 0,4 ; $CH_3OH$)

d) (+)-(6S)-3-Oxo-α-cyclocitralneopentylglykolacetal
$[\alpha]_D^{20}$ = + 49,2° (C = 1 ; $CHCl_3$)
(—)-(6R)-3-Oxo-α-cyclocitralneopentylglykolacetal
$[\alpha]_D^{20}$ = — 48,0° (C = 1 ; $CHCl_3$)

## Beispiel 9

Reracemisierung des nicht benötigten optisch aktiven Ketons

Eine Lösung von 300 mg (+)-3-oxo-α-cyclocitralpropylenglykolacetal in 5 ml Methanol wurde mit 50 mg Natriummethylat versetzt und danach 3 Stunden (h) auf 40 °C erhitzt. Anschließend wurde das Reaktionsgemisch mit 2 ml einer 10 %igen wäßrigen $NH_4Cl$-Lösung versetzt und mit Methyl-tert.-butylether extrahiert. Nach dem Trocknen mit $Na_2SO_4$ und Verdampfen des Lösungsmittels verblieb ein blaßgelbes Öl, dessen spektroskopische Daten mit denen der Ausgangsverbindung identisch waren. Die optische Drehung betrug noch $[\alpha]_D^{20}$ = + 7°.

Das erhaltene racemische 3-Oxo-α-cyclocitralpropylenglykolacetal kann erneut gemäß Beispiel 1a zur Herstellung von (+)- bzw. (—)-3-Oxo-α-cyclocitralpropylenglykolacetalen verwendet werden.

## Beispiel 10

a) Herstellung von optisch aktivem cis- und trans-(6S)-3-Hydroxy-α-cyclocitralpropylenglykolacetal

Eine Lösung von 2,1 g (9 mmol) (+)-(6S)-3-Oxo-α-cyclocitralpropylenglykolacetal in 40 ml Methanol wurde mit 4 g $CeCl_3 \cdot 7H_2O$ versetzt, die Lösung auf — 5 °C gekühlt und unter Rühren portionsweise 400 mg $NaBH_4$ zugefügt. Anschließend wurde das Reaktionsgemisch noch 1 h bei — 5 °C gerührt, dann innerhalb von 0,5 h auf Raumtemperatur (RT) gebracht und noch 1 h bei RT gehalten. Nach dem Zersetzen mit einer Lösung von 1,2 Natriumacetat in 20 ml Wasser wurde 5 × mit je 15 ml Methylenchlorid extrahiert, die organische Phase mit 15 ml Wasser gewaschen und mit $MgSO_4$ getrocknet. Nach dem Abdampfen des Lösungsmittels verblieben 2,10 g eines farblosen Öls, das aus einem 15 : 85 Gemisch der cis/trans-Isomeren von 3-Hydroxy-α-cyclocitralpropylenglykolacetal bestand. Die Ausbeute betrug 100 % der Theorie. Die diastereomeren 3-Hydroxy-acetale wurden durch Säulenchromatographie an Kieselgel

getrennt.

(+)-cis-(3R,6S)-3-Hydroxy-α-cyclocitralpropylenglykolacetal
$[\alpha]_D^{20}$ = + 115,8 (C = 0,3 ; $CH_3OH$)

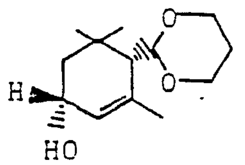

(+)-trans-(3R,6S)-3-Hydroxy-α-cyclocitralpropylenglykolacetal
$[\alpha]_D^{20}$ = + 100,8 (C = 1,0 ; $CH_3OH$)

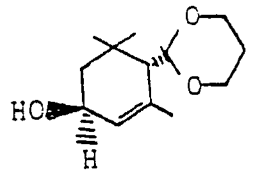

Analog zu Beispiel 10a wurden noch folgende optisch aktiven 3-Hydroxy-α-cyclocitralacetale hergestellt.

b) (—)-trans-(3S,6S)-3-Hydroxy-α-cyclocitralneopentylglykolacetal

$[\alpha]_D^{20}$ = — 65,4° (C = 1 ; $CH_3OH$)

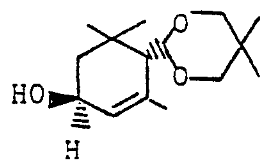

(+)-trans-(3R,6R)-3-Hydroxy-α-cyclocitralneopentylglykolacetal
$[\alpha]_D^{20}$ = + 64,8° (C = 1 ; $CH_3OH$)

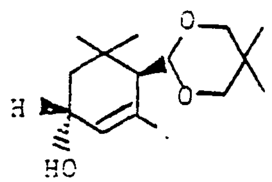

c) (—)-trans-(3S,6S)-3-Hydroxy-α-cyclocitralethylenglykolacetal

$[\alpha]_D^{20}$ = 131,5° (C = 1 ; $CH_3OH$)

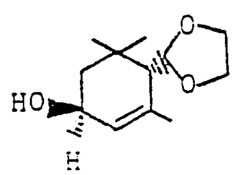

(+)-trans-(3R,6R)-3-Hydroxy-α-cyclocitralethylenglykolacetal
$[\alpha]_D^{20}$ = + 131,2° (C = 1 ; $CH_3OH$)

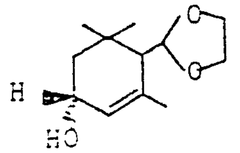

Beispiel 11

Herstellung von (+)-trans-(3S,6S)-3-Hydroxy-α-cyclocitral

Eine Lösung von 500 mg (2,2 mmol) (+)-trans-(3S,6S) 3-Hydroxy-α-cyclocitralpropylenglykolacetal in 10 ml 65 %igem wäßrigem Aceton wurde mit 10 mg p-Toluolsulfonsäure versetzt und das Reaktionsgemisch 12 h bei RT stehengelassen. Anschließend wurde mit 25 ml Wasser verdünnt und 3 × mit je 5 ml Methyl-tert.-butylether extrahiert. Die vereinigten Etherphasen wurden mit 2 ml Wasser gewaschen, mit $MgSO_4$ getrocknet und am Rotationsverdampfer eingeengt. Die Ausbeute betrug 295 mg entsprechend 80 % der Theorie.

$[\alpha]_D^{20} = + 421°/C = 0,5$ ; $CH_3OH)$

Beispiel 12

Herstellung von (—)-(3S)-3-Hydroxy-β-cyclocitral

Eine Lösung von 540 mg (3,2 mmol) (+)-trans-(3S,6S)-3-Hydroxy-α-cyclocitral in 1,5 ml Ethanol wurde bei RT mit 0,3 ml 2 %iger KOH versetzt. Nach einstündigem Rühren wurden 20 ml $CH_2Cl_2$ zugefügt, die organische Phase erst mit 2 ml gesättigter $NH_4Cl$-Lösung und dann mit $H_2O$ gewaschen. Nach Trocknen mit $MgSO_4$ und Einengen verblieben 450 mg eines blaßgelben Öls, das neben 90,7 % des gewünschten Produktes 9,3 % des Ausgangsmaterials enthielt.

$[\alpha]_D^{20} = — 34,8°$ (C = 0,85, $CH_3OH$)

Die entsprechenden optischen Antipoden der gemäß den Beispielen 3-5 hergestellten Verbindungen werden erhalten, wenn als Ausgangsmaterial das entsprechende (—)-3-Oxo-α-cyclocitralacetal für die weitere Umsetzung eingesetzt wird.

**Patentansprüche**

1. 3-Hydroxy-α-cyclocitralderivate der allgemeinen Formel I

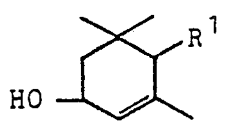

(I)

in der $R^1$ für

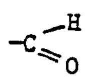 oder 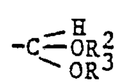

steht, worin $R^2$ und $R^3$ gleich oder verschieden sein können und jeweils eine lineare oder verzweigte Alkylgruppe mit 1 bis 6 C-Atomen bedeuten oder aber $R^2$ und $R^3$ zusammen für eine Ethylengruppe oder eine Propylengruppe stehen, die durch Methylgruppen oder Ethylgruppen substituiert sein können.

2. Optisch aktive α-Cyclocitralderivate der allgemeinen Formeln A und B

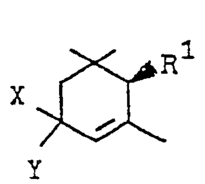

A

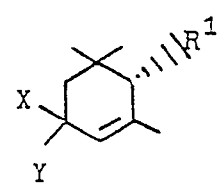

B

in denen $R^1$ für

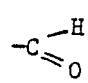

steht, wenn X für Wasserstoff und Y für eine OH-Gruppe steht, oder aber $R^1$ für

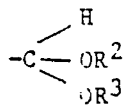

steht, worin $R^2$ und $R^3$ gleich oder verschieden sein können und jeweils eine lineare oder verzweigte Alkylgruppe mit 1 bis 6 C-Atomen bedeuten oder $R^2$ und $R^3$ zusammen für eine Ethylengruppe oder eine Propylengruppe stehen, die durch Methylgruppen oder Ethylgruppen substituiert sein können, und X und Y zusammen für Sauerstoff stehen oder X für Wasserstoff steht, wenn Y eine OH-Gruppe bedeutet.

3. Optisch aktive 3-Hydroxy-α-cyclocitralderivate der allgemeinen Formeln A und B gemäß Anspruch 2, in denen X für Wasserstoff steht, wenn Y eine OH-Gruppe bedeutet.

4. Optisch aktive 3-Oxo-α-cyclocitralderivate der allgemeinen Formeln A und B (A III und B III) gemäß Anspruch 2, in denen X und Y zusammen für Sauerstoff stehen.

5. Verfahren zur Herstellung von 3-Hydroxy-α-cyclocitralderivaten der allgemeinen Formel I

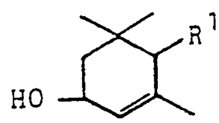

(I)

worin $R^1$, $R^2$ und $R^3$ die in Anspruch 1 angegebene Bedeutung haben, dadurch gekennzeichnet, daß man

a) das entsprechende Trimethylcyclohexenacetal der allgemeinen Formel II

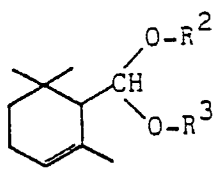

(II)

mit einem geeigneten Oxidationsmittel zu dem 3-Oxo-α-cyclocitralacetal der allgemeinen Formel III

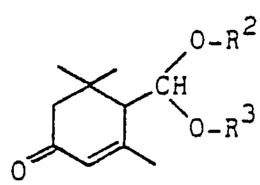

(III)

oxidiert,

b) dieses mit einem geeigneten Reduktionsmittel zu dem 3-Hydroxy-α-cyclocitralacetal der allgemeinen Formel Ia reduziert

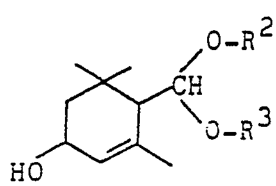

(Ia)

und dieses gegebenenfalls

c) in Gegenwart von organischen oder anorganischen Säuren in wäßrigen organischen Lösungsmitteln zu dem 3-Hydroxy-α-cyclocitral der Formel Ib

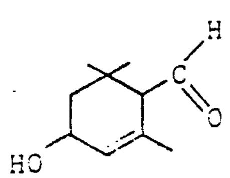

(Ib)

hydrolysiert.

6. Verfahren gemäß Anspruch 2, dadurch gekennzeichnet, daß man im Verfahrensschritt a) das Trimethylcyclohexenacetal der Formel II mit Sauerstoff und tert.-Butylhydroperoxid in Gegenwart von Rhodiumverbindungen als geeignetem Oxidationsmittel oxidiert.

7. Verfahren gemäß Anspruch 3, dadurch gekennzeichnet, daß man die Oxidation in Gegenwart von Rhodiumtrichloridhydrat oder Rhodiumtristriphenylphosphinchlorid als Rhodiumverbindung durchführt.

8. Verfahren gemäß Anspruch 2, dadurch gekennzeichnet, daß man im Verfahrensschritt a) das Trimethylcyclohexenacetal der Formel II mit tert.-Butylhydroperoxid in Gegenwart von Cu(II) Acetylacetonat als geeignetem Oxidationsmittel oxidiert.

9. Verfahren gemäß Anspruch 2, dadurch gekennzeichnet, daß man im Verfahrensschritt a) das Trimethylcyclohexenacetal der Formel II mit Di-tert.-butylchromat als geeignetem Oxidationsmittel oxidiert.

10. Verfahren gemäß Anspruch 2, dadurch gekennzeichnet, daß man im Verfahrensschritt b) das 3-Oxo-α-cyclocitralacetal mit Natriumborhydrid in Gegenwart von Cer(III)-verbindungen oder mit Diisobutylaluminiumhydrid oder mit Lithium-tri-tert.-butoxyaluminiumhydrid als geeignetem Reduktionsmittel reduziert.

11. Verfahren gemäß Anspruch 2, dadurch gekennzeichnet, daß man im Verfahrensschritt c) das 3-Hydroxy-α-cyclocitralacetal der Formel III mit p-Toluolsulfonsäure in etwa 70 vol.-%igem wäßrigem Aceton hydrolysiert.

12. Verwendung von 3-Hydroxy-α-cyclocitral-acetalen der allgemeinen Formel Ia zur Herstellung von Safranal der Formel IV

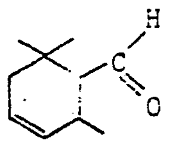

(IV)

durch Hydrolyse in Gegenwart von Säuren in einem geeigneten wasserfreien Lösungsmittel.

13. Verwendung von 3-Hydroxy-α-cyclocitral der Formel Ib

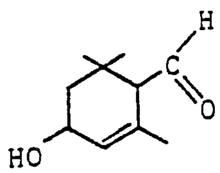

(Ib)

zur Herstellung von 3-Hydroxy-β-cyclocitral der Formel V

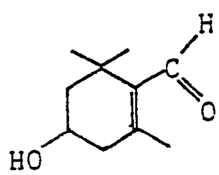

(V)

durch Isomerisieren in Gegenwart von Alkalihydroxiden oder Alkalialkoxiden in geeigneten organischen Lösungsmitteln.

14. Verfahren zur Herstellung von optisch aktiven 3-Oxo-α-cyclocitralacetalen der allgemeinen Formeln A III und B III

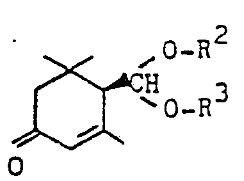

A III

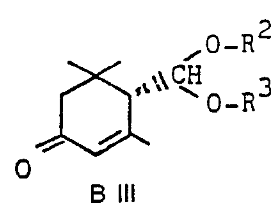

B III

dadurch gekennzeichnet, daß man Gemische der entsprechenden racemischen 3-Oxo-α-cyclocitralacetale durch Chromatographie an geeigneten chiralen Adsorbentien in die optisch aktiven Verbindungen trennt.

15. Verfahren zur Herstellung von optisch aktiven 3-Oxo-α-cyclocitralacetalen der allgemeinen Formeln A III und B III gemäß Anspruch 14, dadurch gekennzeichnet, daß man Gemische der entsprechenden racemischen 3-Oxo-α-cyclocitralacetale durch Chromatographie an triacetylierter Cellulose mit niederen Alkanolen als Elutionsmittel trennt.

16. Verfahren zur Herstellung von optisch aktiven α-Cyclocitralderivaten der allgemeinen Formeln A und B

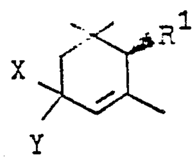

A

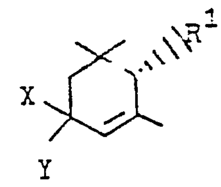

B

worin X und Y sowie $R^1$, $R^2$ und $R^3$ die in Anspruch 1 angegebene Bedeutung haben, dadurch gekennzeichnet, daß man

a) das entsprechende Trimethylcyclohexenacetal der allgemeinen Formel II

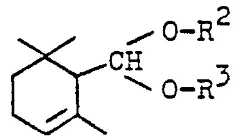

(II)

mit einem geeigneten Oxidationsmittel zu 3-Oxo-α-cyclocitralacetalen der allgemeinen Formel III

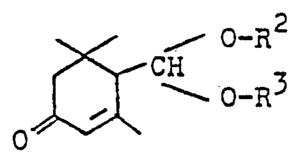

(III)

oxidiert,

aa) dieses gemäß dem Verfahren nach den Ansprüchen 14 oder 15 in die optisch aktiven 3-Oxo-α-cyclocitralacetale der Formeln A III und B III

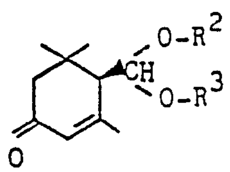

A III

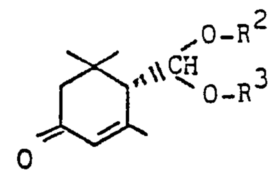

B III

trennt,

b) diese gegebenenfalls mit einem geeigneten Reduktionsmittel stereoselektiv zu optisch aktiven trans- oder cis-3-Hydroxy-α-cyclocitralacetalen der allgemeinen Formeln A'I und B'I reduziert

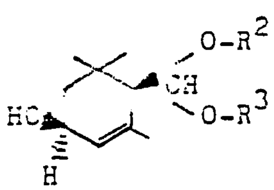

cis-A'I

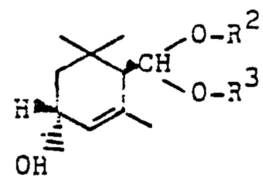

trans-A'I

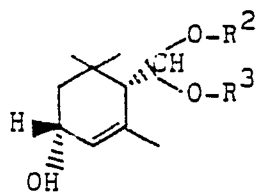

cis-B'I

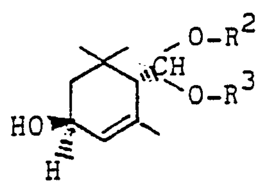

trans-B'I

und diese gegebenenfalls

c) in Gegenwart von organischen oder anorganischen Säuren in wäßrigen organischen Lösungsmitteln zu optisch aktivem 3-Hydroxy-α-cyclocitral der Formeln A''I und B''I

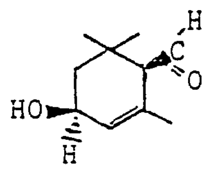

cis-A"I

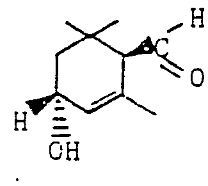

trans-A"I

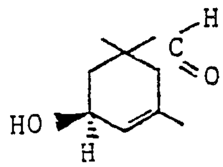

trans-B"I

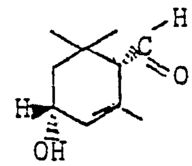

cis-B"I

hydrolysiert.

17. Verwendung von optisch aktiven 3-Hydroxy-α-cyclocitralen der Formel A"I bzw. B"I

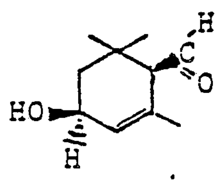

cis-A"I

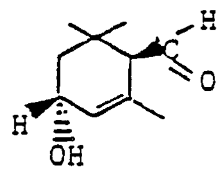

trans-A"I

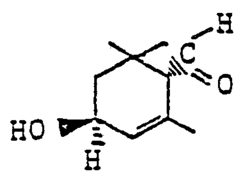

trans-B"I

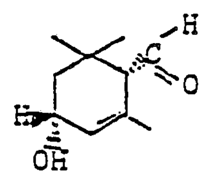

cis-B"I

zur Herstellung von optisch aktivem 3-Hydroxy-β-cyclocitral der Formeln Va und Vb

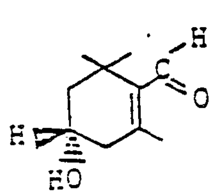

(Va)

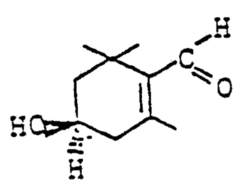

(Vb)

durch Isomerisieren in Gegenwart von Alkalihydroxiden oder Alkalialkoxiden in geeigneten organischen Lösungsmitteln.

**Claims**

1. A 3-hydroxy-α-cyclocitral derivative of the formula I

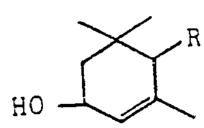

(I)

where $R^1$ is

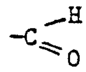 or 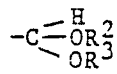

wherein $R^2$ and $R^3$ can be identical or different and are each straightchain or branched alkyl of 1 to 6 carbon atoms, or $R^2$ and $R^3$ together form an ethylene or propylene group which can be substituted by methyl or ethyl.

2. Optically active -cyclocitral derivatives of the formulae A and B

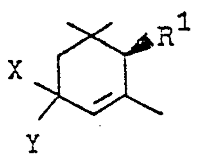 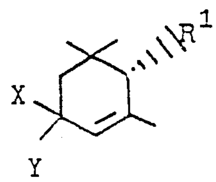

A B

where $R^1$ is

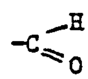

if X is hydrogen and Y is OH, but is otherwise

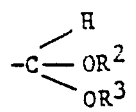

where $R^2$ and $R^3$ can be identical or different and are each straight-chain or branched alkyl of 1 to 6 carbon atoms, or $R^2$ and $R^3$ together form an ethylene or propylene group which can be substituted by methyl or ethyl, and X and Y together are oxygen, or X is hydrogen if Y is OH.

3. Optically active 3-hydroxy-α-cyclocitral derivatives of the formulae A and B as claimed in claim 2, wherein X is hydrogen if Y is OH.

4. Optically active 3-oxo-α-cyclocitral derivatives of the formulae A and B (A III and B III) as claimed in claim 2, wherein X and Y together are oxygen.

5. A process for the preparation of a 3-hydroxy-α-cyclocitral derivative of the formula I

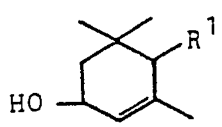

(I)

where $R^1$, $R^2$ and $R^3$ have the meanings given in claim 1, wherein

a) the corresponding trimethylcyclohexene acetal of the formula II

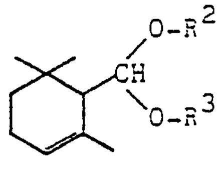

(II)

is oxidized with a suitable oxidizing agent to the 3-oxo-α-cyclocitral acetal of the formula III

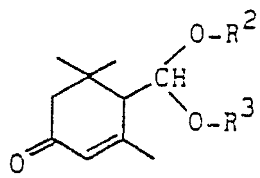

(III)

b) this is reduced with a suitable reducing agent to the 3-hydroxy- -cyclocitral acetal of the formula Ia

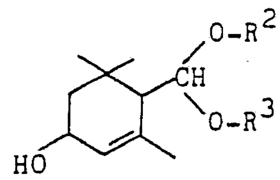

(Ia)

and, if required

c) this is hydrolyzed in the presence of an organic or inorganic acid in aqueous organic solvent to give the 3-hydroxy-α-cyclocitral of the formula Ib

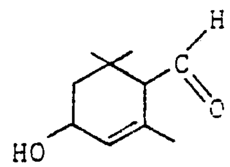

(Ib)

6. A process as claimed in claim 2, wherein, in step a), the trimethylcyclohexene acetal of the formula II is oxidized with oxygen and tert.-butyl hydroperoxide in the presence of a rhodium compound as a suitable oxidizing agent.

7. A process as claimed in claim 3, wherein the oxidation is carried out in the presence of rhodium trichloride hydrate or rhodium-tristriphenylphosphonium chloride as the rhodium compound.

8. A process as claimed in claim 2, wherein in step a), the trimethylcyclohexene acetal of the formula II is oxidized with tert.-butyl hydroperoxide in the presence of Cu(II) acetylacetonate as a suitable oxidizing agent.

9. A process as claimed in claim 2, wherein, in step a), the trimethylcyclohexene acetal of the formula II is oxidized with di-tert.-butyl chromate as a suitable oxidizing agent.

10. A process as claimed in claim 2, wherein, in step b), the 3-oxo-α-cyclocitral acetal is reduced with sodium borohydride in the presence of a cerium(III) compound or with diisobutyl-aluminum hydride or with lithium tri-tert.-butoxy-aluminium hydride as a suitable reducing agent.

11. A process as claimed in claim 2, wherein, in step c), the 3-hydroxy-α-cyclocitral acetal of the formula III is hydrolyzed with p-toluenesulfonic acid in about 70 % strength by volume aqueous acetone.

12. Use of a 3-hydroxy-α-cyclocitral acetal of the formula Ia for the preparation of safranal of the formula IV

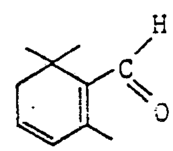

(IV)

by hydrolysis in the presence of an acid in a suitable anhydrous solvent.

13. Use of a 3-hydroxy-α-cyclocitral of the formula Ib

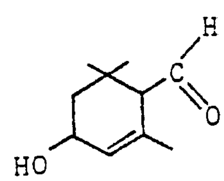

(Ib)

for the preparation of 3-hydroxy-β-cyclocitral of the formula V

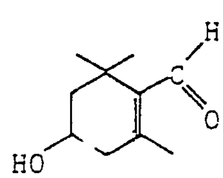

(V)

by isomerization in the presence of an alkali metal hydroxide or alkali metal alkoxide in a suitable organic solvent.

14. A process for the preparation of opticall active 3-oxo- -cyclocitral acetals of the formulae A III and B III

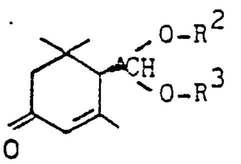

A III

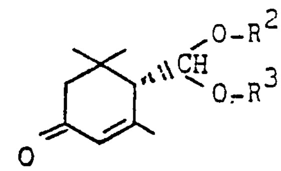

B III

wherein a mixture of the corresponding racemic 3-oxo-α-cyclocitral acetals is separated into the optically active compounds by chromatography over a suitable chiral adsorbent.

15. A process for the preparation of optically active 3-oxo-α-cyclocitral acetals of the formulae A III and B III as claimed in claim 14, wherein a mixture of the corresponding racemic 3-oxo-α-cyclocitral acetals is separated by chromatography over cellulose triacetate, using a lower alkanol as an eluting agent.

16. A process for the preparation of optically active α-cyclocitral derivatives of the formulae A and B

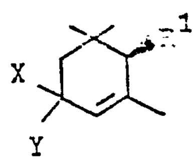

A

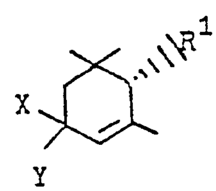

B

where X, Y, $R^1$, $R^2$ and $R^3$ have the meanings given in claim 1, wherein

a) the corresponding trimethylcyclohexene acetal of the formula II

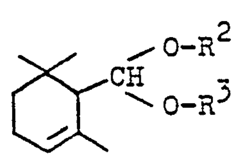

(II)

is oxidized vith a suitable oxidizing agent to 3-oxo-α-cyclocitral acetal of the formula III

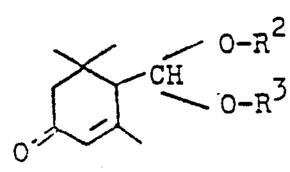

(III)

aa) this is separated into the optically active 3-oxo-α-cyclocitral acetals of the formulae A III and B III

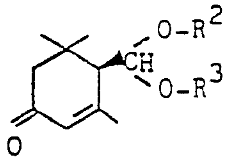

A III

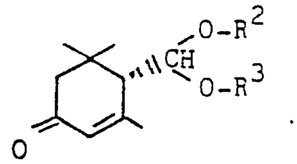

B III

by the process according to claim 14 or 15,

b) these are, if desired, reduced stereoselectively with a suitable reducing agent to optically active trans- or cis-3-hydroxy-α-cyclocitral acetals of the formulae A'I and B'I

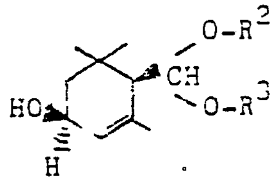

cis-A'I

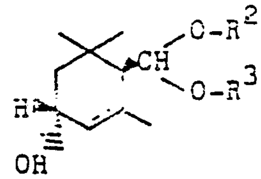

trans-A'I

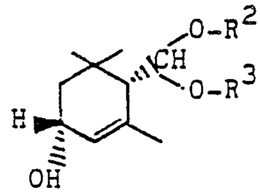

cis-B'I

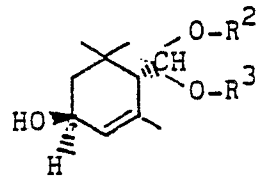

trans-B'I

and, if desired,

c) these are hydrolyzed in the presence of an organic or inorganic acid in an aqueous organic solvent to give the optically active 3-hydroxy-α-cyclocitrals of the formulae A"I and B"I

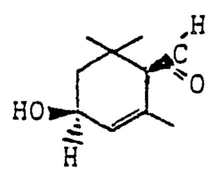

cis-A"I

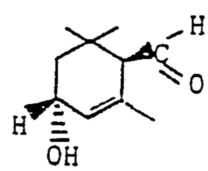

trans-A"I

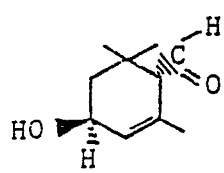

trans-B"I

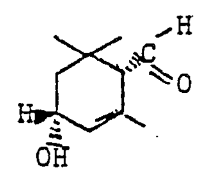

cis-B"I

17. Use of an optically active 3-hydroxy-α-cyclocitral of the formula A"I or B"I

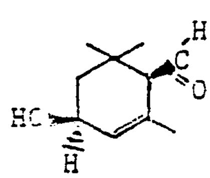

cis-A"I

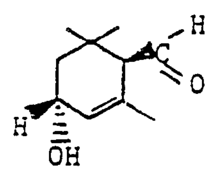

trans-A"I

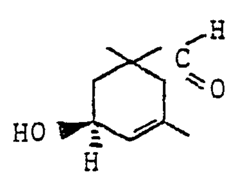

trans-B"I

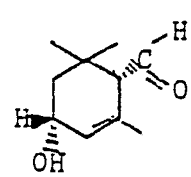

cis-B"I

for the preparation of an optically active 3-hydroxy- -cyclocitral of the formula Va or Vb

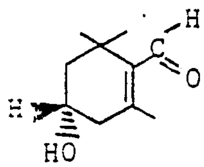
(Va)

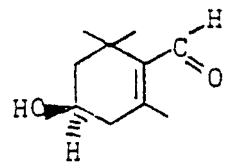
(Vb)

by isomerization in the presence of an alkali metal hydroxide or alkali metal alkoxide in a suitable organic solvent.

**Revendications**

1. Dérivés de 3-hydróxy-$\alpha$-cyclocitral de formule générale I

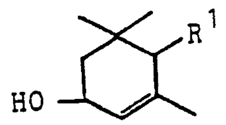

dans laquelle $R^1$ est mis pour

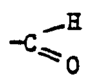 ou 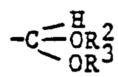

$R^2$ et $R^3$ pouvant être semblables ou différents et représentant chacun un groupe alkyle à 1-6 atomes de C à chaîne droite ou ramifiée, ou $R^2$ et $R^3$ pouvant aussi former ensemble un groupe éthylène ou un groupe propylène qui peuvent être substitués par des groupes méthyle ou des groupes éthyle.

2. Dérivés d'$\alpha$-cyclocitral optiquement actifs de formules générales A et B

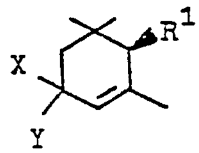
A

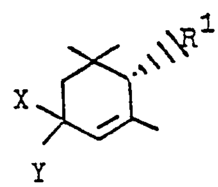
B

dans lesquelles $R^1$ est mis pour

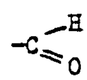

lorsque X représente un atome d'hydrogène et que Y représente un groupe OH, ou bien $R^1$ est mis pour

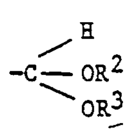

$R^2$ et $R^3$ pouvant être semblables ou différents et représentant chacun un groupe alkyle à 1-6 atomes de C à chaîne droite ou ramifiée, ou $R^2$ et $R^3$ pouvant aussi former ensemble un groupe éthylène ou un groupe propylène qui peuvent être substitués par des groupes méthyle ou des groupes éthyle, et X et Y forment ensemble un atome d'oxygène ou X est mis pour un atome d'hydrogène lorsque Y représente un groupe OH.

3. Dérivés de 3-hydroxy-$\alpha$-cyclocitral optiquement actifs de formules générales A et B selon la revendication 2, dans lesquels X est mis pour un atome d'hydrogène lorsque Y représente un groupe OH.

4. Dérivés de 3-oxo-$\alpha$-cyclocitral optiquement actifs de formules générales A et B (A III et B III) selon la revendication 2, dans lesquels X et Y forment ensemble un atome d'oxygène.

5. Procédé de préparation de dérivés de 3-hydroxy-$\alpha$-cyclocitral de formule générale I

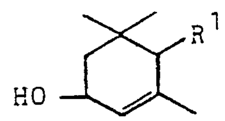

(I)

dans laquelle $R^1$, $R^2$ et $R^3$ ont les significations données dans la revendication 1, caractérisé en ce que :

a) on oxyde le triméthylcyclohexène-acétal correspondant de formule générale II

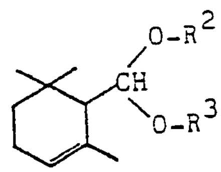

(II)

avec un oxydant approprié pour obtenir le 3-oxo-α-cyclocitralacétal de formule générale III

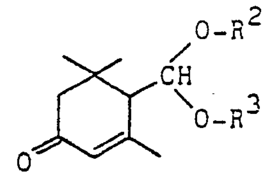

(III)

b) on réduit celui-ci avec un réducteur approprié pour obtenir le 3-hydroxy-α-cyclocitralacétal de formule générale Ia

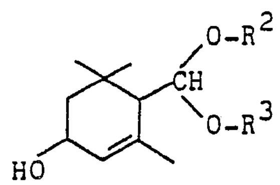

(Ia)

c) et, le cas échéant, on hydrolyse celui-ci en présence d'acides organiques ou minéraux dans des solvants organiques aqueux pour obtenir le 3-hydroxy-α-cyclocitral de formule Ib

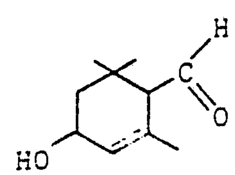

(Ib)

6. Procédé selon la revendication 5, caractérisé en ce que, dans la phase opératoire a), on oxyde le triméthylcyclohexène-acétal de formule II avec, en tant qu'oxydant approprié, de l'oxygène et de l'hydroperoxyde de butyle tertiaire en présence de composés du rhodium.

7. Procédé selon la revendication 6, caractérisé en ce qu'on effectue l'oxydation en présence d'hydrate de trichlorure de rhodium ou de chlorure de tristriphénylphosphine de rhodium en tant que composé du rhodium.

8. Procédé selon la revendication 5, caractérisé en ce que, dans la phase opératoire a), on oxyde le triméthylcyclohexène-acétal de formule II avec, en tant qu'oxydant approprié, de l'hydroperoxyde de butyle tertiaire en présence d'acétylacétonate cuivrique.

9. Procédé selon la revendication 5, caractérisé en ce que, dans la phase opératoire a), on oxyde le triméthylcyclohexène-acétal de formule II avec, en tant qu'oxydant approprié, du chromate de dibutyle tertiaire.

10. Procédé selon la revendication 5, caractérisé en ce que, dans la phase opératoire b), on réduit le 3-oxo-α-cyclocitralacétal avec, en tant que réducteur approprié, du borhydrure de sodium en présence de composés céreux, de l'hydrure de diisobutyl-aluminium ou de l'hydrure de lithium-tri-tert.-butoxyaluminium.

11. Procédé selon la revendication 5, caractérisé en ce que dans la phase opératoire c), on hydrolyse le 3-hydroxy-α-cyclocitralacétal de formule III avec de l'acide p-toluènesulfonique dans l'acétone aqueux à 70 % en volume environ.

12. Utilisation de 3-hydroxy-α-cyclocitralacétal de formule générale Ia pour la préparation de safranal de formule IV

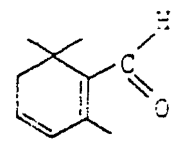

(IV)

par hydrolyse en présence d'acides dans un solvant anhydre approprié.

13. Utilisation de 3-hydroxy-α-cyclocitral de formule Ib

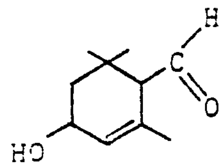

(Ib)

pour la préparation de 3-hydroxy-β-cyclocitral de formule V

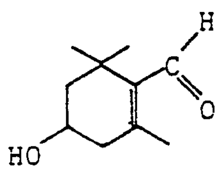

(V)

par isomérisation en présence d'hydroxydes alcalins ou d'alcoxydes alcalins dans des solvants organiques appropriés.

14. Procédé de préparation de 3-oxo-α-cyclocitralacétals optiquement actifs de formules générales A III et B III

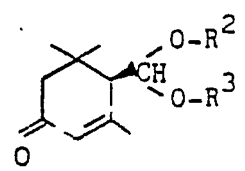

A III

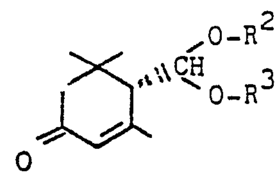

B III

caractérisé en ce qu'on sépare en les composés optiquement actifs des mélanges des 3-oxo-α-cyclocitralacétals racémiques correspondants par chromatographie sur des adsorbants chiraux appropriés.

15. Procédé de préparation de 3-oxo-α-cyclocitralacétals optiquement actifs de formules générales A III et B III selon la revendication 14, caractérisé en ce qu'on sépare des mélanges des 3-oxo-α-cyclocitralacétals racémiques correspondants par chromatographie sur une cellulose triacétylée, en utilisant des alcanols inférieurs comme éluant.

16. Procédé de préparation de dérivés d'α-cyclocitral optiquement actifs de formules générales A et B

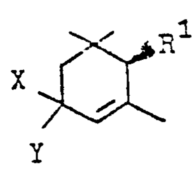

A

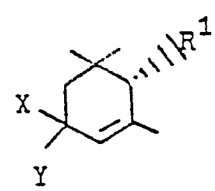

B

dans lesquelles X et Y, ainsi que $R^1$, $R^2$ et $R^3$ ont les significations données dans la revendication 1, caractérisé en ce que :

a) on oxyde le triméthylcyclohexène-acétal correspondant de formule générale II

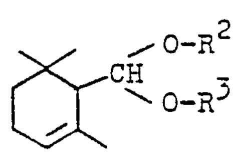

(II)

avec un oxydant approprié pour obtenir des 3-oxo-α-cyclocitralacétals de formule générale III

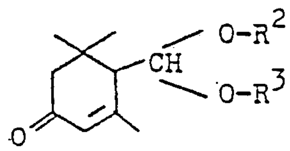

(III)

aa) on sépare ceux-ci, suivant le procédé selon la revendication 14 ou 15, en les 3-oxo-α-cyclocitralacétals optiquement actifs de formules A III et B III

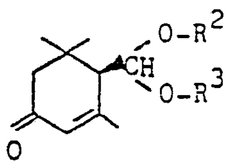

A III

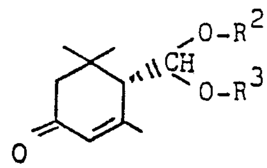

B III

b) on réduit éventuellement ceux-ci stéréosélectivement, avec un réducteur approprié, en trans- ou cis-3-hydroxy-α-cyclocitralacétals optiquement actifs de formules générales A'I et B'I

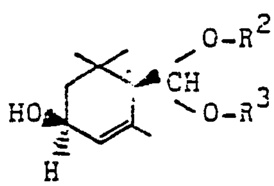

cis-A'I

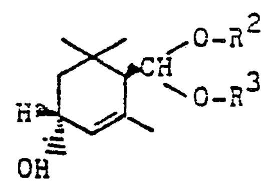

trans-A'I

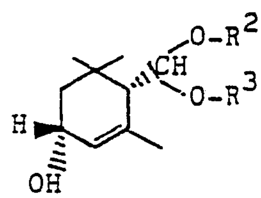

cis-B'I

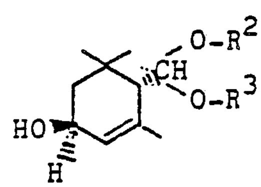

trans-B'I

et le cas échéant,

c) on hydrolyse ceux-ci, en présence d'acides organiques ou minéraux, dans des solvants organiques aqueux, en 3-hydroxy-α-cyclocitrals optiquement actifs de formules A"I et B"I

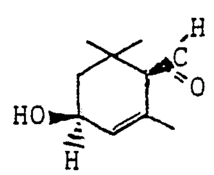

cis-A"I

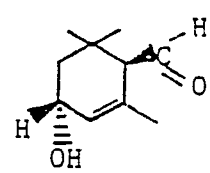

trans-A"I

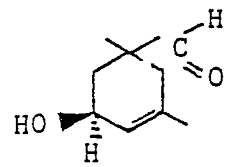

trans-B"I

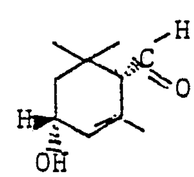

cis-B"I

17. Utilisation de 3-hydroxy-α-cyclocitrals optiquement actifs de formules A"I ou B"I

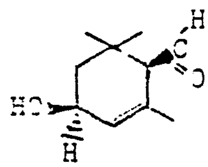

cis-A"I

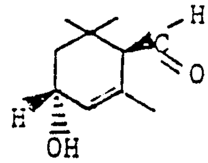

trans-A"I

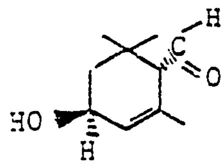

trans-B"I

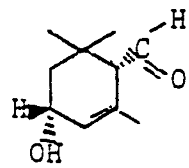

cis-B-"I

pour la préparation de 3-hydroxy-β-cyclocitrals optiquement actifs de formules Va et Vb

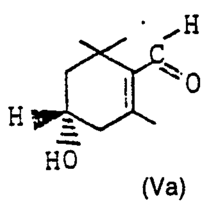

(Va)

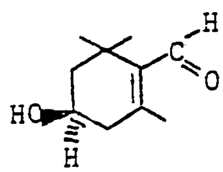

(Vb)

par isomérisation en présence d'hydroxydes alcalins ou d'alcoxydes alcalins dans des solvants organiques appropriés.